# EUROPÄISCHE PATENTSCHRIFT

(11) **EP 2 996 650 B1**
(45) Veröffentlichungstag und Bekanntmachung des Hinweises auf die Patenterteilung: **04.09.2024**
(21) Anmeldenummer: 14729229.6
(22) Anmeldetag: 16.05.2014
(51) Int. Cl.: A61F 13/00

(54) **WUNDPFLEGEVORRICHTUNG ZUR BEHANDLUNG VON WUNDEN MITTELS ATMOSPHÄRISCHEM UNTERDRUCK, AUFWEISEND EIN ÖFFENBARES FENSTER**
WOUND CARE DEVICE FOR TREATING WOUNDS BY MEANS OF SUBATMOSPHERIC PRESSURE, HAVING OPENABLE WINDOW
DISPOSITIF DE SOIN DE PLAIES POUR LE TRAITEMENT DES PLAIES AU MOYEN D'UNE DÉPRESSION ATMOSPHÉRIQUE, COMPRENANT UNE FENÊTRE OUVRABLE

(30) Priorität: 16.05.2013 DE 102013105063; 12.07.2013 DE 102013107399
(43) Veröffentlichungstag der Anmeldung: 23.03.2016
(73) Patentinhaber: BSN medical GmbH, 22761 Hamburg (DE)
(72) Erfinder: Riesinger, Birgit, 48149 Münster (DE)
(74) Vertreter: Cohausz & Florack
(86) Internationale Anmeldenummer: PCT/EP2014/060133
(87) Internationale Veröffentlichungsnummer: WO 2014/184366

(56) Entgegenhaltungen:
- EP-A1- 1 814 609
- EP-B1- 1 814 609
- WO-A1-2012/168298
- US-A- 4 468 227
- US-A- 5 086 763

## Beschreibung

Die Erfindung betrifft eine Wundpflegevorrichtung zur Behandlung von Wunden mittels atmosphärischem Unterdruck, aufweisend ein öffenbares Fenster.

Herkömmliche Systeme und Vorrichtungen zur Behandlung von Wunden mittels atmosphärischem Unterdruck bestehen aus einer gasdichten Wundabdeckung, einem Drainageschlauch, einer extern angeordneten Vakuumpumpe sowie einem Sammelgefäß für die Aufnahme von abgeführten Exsudaten.

Solche Vorrichtungen sind beispielsweise im Patent US7198046 beschrieben. Aus der EP1814609 ist eine Wundpflegevorrichtung zur Behandlung von Wunden mittels atmosphärischem Unterdruck beschrieben. Diese kann optional ein öffenbares Fenster aufweisen. Dieses wird als "gasdichtes Behandlungsfenster" bezeichnet, oder auch als "schwenkbares Verschlusselement".

WO 2012/168298 A1 offenbart eine Wundpflegevorrichtung zur Behandlung von Wunden mittels atmosphärischem Unterdruck, aufweisend ein Wundabdeckungselement, das an der Haut eines Patienten befestigbar ist, sowie eine Anschlussvorrichtung zum Ansaugen von fluiden Medien, wobei das Wundabdeckungselement ein öffenbares Fenster aufweist.

Vorrichtungen zur Behandlung von Wunden mittels atmosphärischem Unterdruck dienen beispielsweise zur postoperativen Behandlung von Inzisionen (hier gilt es, das Nahtwasser abzuführen und so eine schneller und komplikationsärmere Nahtheilung zu ermöglichen), als auch bei der Behandlung von tiefliegenden Ödemen, beispielsweise bei Dekubitus oder Ulcus cruris (hier ist die aktive Akquirierung von Wundflüssigkeit aus der Tiefe Voraussetzung dafür, dass die chronische Wunde überhaupt heilt).

In der Regel kommen in dieser Therapieform relative Unterdrücke zwischen 60 und 200 mm Hg zum Einsatz. Das aus der EP1814609 beschriebene öffenbare Fenster dient dazu, dass ein darunter angeordnetes Wundkontaktelement, beispielsweise ein Absorptionskörper, oder ein sekundär angeordneter Pflegeartikel in den durch die gasdichte Wundabdeckung gebildeten Raum einlegbar bzw. aus dem Raum entnehmbar ist. Auf diese Weise kann besagtes Wundkontaktelement oder besagter Pflegeartikel aus dem Wundraum entnommen werden - beispielsweise zwecks Wundreinigung, oder zwecks Austausch - ohne dass die gesamte Wundpflegevorrichtung - die vorzugsweise auf die um die Wunde angeordnete Haut des Patienten geklebt wird - ausgetauscht werden muss. Dies wiederum vermindert traumatische Eingriffe während der Wundversorgung, und ist auch aus Kostenaspekten vorteilhaft.

Aufgrund der atmosphärischen Verhältnisse ist es erforderlich, dass besagtes öffenbares Fenster im geschlossenen Zustand gasdicht schließt. Dies ist nicht trivial, da Luft ein sehr gering viskoses Medium ist. Darüber hinaus erfordern viele Arten der gasdichten Verbindungen eine ebenfalls starke physische Verbindung - was im Wundkontakt unerwünscht ist, da solche starken physischen Verbindungen einerseits eine gewisse Bauhöhe und ein gewisses Gewicht aufweisen, und andererseits ein gewisse Resilienz beim Öffnen bzw. Schließen aufweisen - in anderen Worten: das Öffnen bzw. Schließen erfolgt mit händisch ausgeführter Kraft.

Dies ist im vorliegenden Kontext jedoch höchst unerwünscht, da das Ausüben von Kräften im Wundbereich häufig zu Schmerzeinwirkungen beim Patienten führen kann. Sollte beispielsweise das Schließen eines Behandlungsfensters Kraft erfordern, so wird der Patient dies als traumatischen Eingriff erleben - insbesondere bei den Indikationen Narbe, Dekubitus und Ulcus cruris. Aufgabe der vorliegenden Erfindung ist es daher, eine Vakuum-Wundversorgung bereitzustellen, die den Austausch von Wundkontaktelementen oder Pflegeartikeln ermöglicht, ohne dass sich dabei die genannten Nachteile einstellen.

### AUSFÜHRLICHE BESCHREIBUNG DER ERFINDUNG

Erfindungsgemäß ist eine Wundpflegevorrichtung gemäß Anspruch 1 zur Behandlung von Wunden mittels atmosphärischem Unterdruck im Wundbereich vorgesehen.

Besagtes öffenbares Fenster weist eine Vielzahl von Vorteilen auf. So ermöglicht es
- ein erleichtertes Spülen oder Reinigen der darunter liegenden Wunde,
- eine Behandlung derselben mit Pharmazeutika oder Pflegeprodukten,
- den atraumatischen Wechsel einer Wundauflage oder eines Absorptionskörpers
- das Inspizieren und Begutachten der Wunde, und
- eine Probennahme, beispielsweise eines Abstriches,
ohne dass die gesamte Wundpflegevorrichtung abgenommen werden muss.

Der Begriff "öffenbares Fenster" umfasst sowohl komplett durchsichtige Fenster, als auch Fenster, die nicht durchsichtig oder mit einem nicht durchsichtigen Material unterlegt sind, als auch Fenster, die einen durchsichtigen Bereich aufweisen.

Bevorzugt ist dabei vorgesehen, dass das Wundabdeckungselement dem anatomischen Relief einer gegebene Körperposition entsprechend vorgeformt ist. Dies kann beispielsweise sinnvoll sein, wenn das Wundabdeckungselement im Bereich der Armbeuge, der Hüfte oder des Knies angelegt werden soll. Beispielsweise mittels Tiefziehverfahren lässt sich dabei insbesondere das Wundabdeckungselement dem anatomischen Relief der genannten Körperpositionen entsprechend vorformen.

Ebenso bevorzugt ist dabei vorgesehen, dass das Wundabdeckungselement dergestalt ausgebildet ist, dass es dem Bewegungsablauf gegebene Körperposition durch folgen kann. Hierfür kann beispielsweise das Wundabdeckungselement und auch der gasdichte Verschluss elastisch ausgebildet sein. Ebenso kann eine Plissierung oder ein oder mehrere Dehnungsbälge vorgesehen sein.

Wichtig bei den vorgenannten Ausführungsformen ist insbesondere, dass der genannte gasdichte Verschluss auch unter diesen erschwerten Bedingungen seine Gasdichtigkeit beibehält.

Besagter gasdichter Verschluss ist dabei bevorzugt in Form eines Leistenverschlusses ausgeführt. Unter dem Begriff "Leistenverschluss" werden im Folgenden sämtliche Arten von öffenbaren, formschlüssigen Verschlüssen subsummiert, die nach dem Nut/Feder-Prinzip funktionieren.

Hier sind auf den beiden per Verschluss miteinander zu verbindenden Werkstücken Lippen vorgesehen, die beim Verschließen formschlüssig ineinander greifen. Besagte Lippen bestehen bevorzugt aus elastischen Materialien, beispielsweise Gummi, Kautschuk, Polyethylen oder Polypropylen.

Solche Verschlüsse sind beispielsweise aus wiederverschließbaren Handgepäckbeuteln für den Transport von Kosmetika im Luftverkehr, wiederverschließbaren Vorratsbeuteln für die Tiefkühl-Lagerung, oder wiederverschließbaren Vorratsgefäßen ("Tupperdosen") bekannt.

Die besagten Leistenverschlüsse sind auch unter den Pseudonymen "Ziplock", "Minigrip" oder Gleitverschluss bekannt. In letzterem Fall kann ein Gleiter vorgesehen sein, der ähnlich wie bei einem Reißverschluss entlang der zu verschließenden Lippen gleitet und sie in die Schließposition drängt.

Ferner fallen auch sogenannte Wulstverschlüsse, wie sie beispielsweise in der WO03013976A1 beschrieben sind, unter den Begriff "Leistenverschluss".

Wichtig ist, dass besagte Verschlüsse geeignet sind, besagtes öffenbares Fenster im geschlossenen Zustand gasdicht zu schließen, ferner eine geringe Bauhöhe und ein geringes Gewicht aufweisen bzw. erfordern, und überdies eine geringe Kraft beim Schließen bzw. Öffnen erfordern. Auf diese Weise kann das Öffnen bzw. Schließen ohne Schmerzeinwirkungen ausgeübt werden.

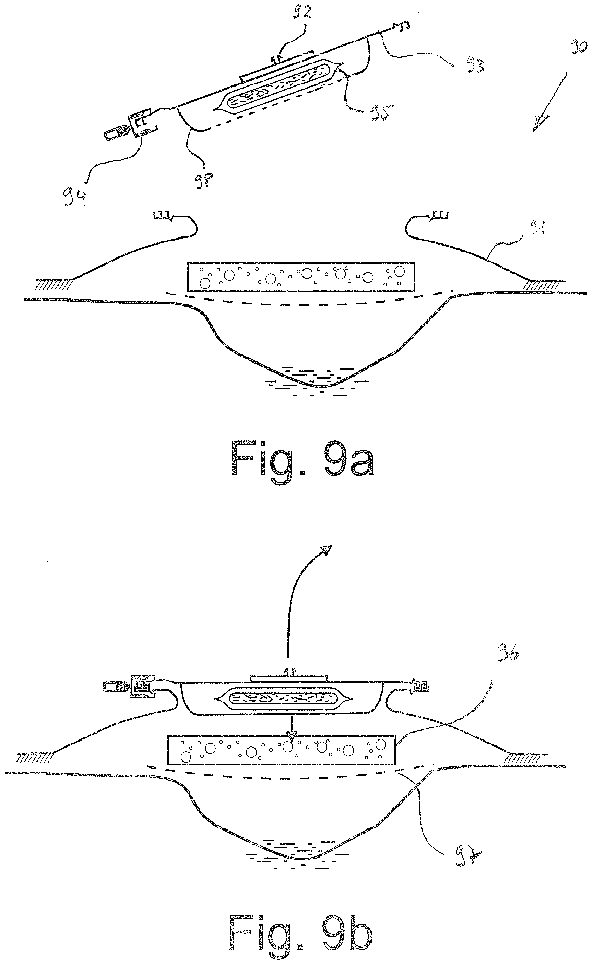

Letzteres läßt sich beispielsweise durch eine Ausführung mit einem Gleiter (siehe Fig. 9 und Beschreibung) oder eine Ausführung mit einer Hintergriffsmöglichkeit (siehe Fig, 4 und Beschreibung) bewerkstelligen.

Alternativ zu obiger Ausführungsform kann vorgesehen sein, dass besagter gasdichter Verschluss in Form eines Magnetverschlusses ausgeführt ist. Hierzu können insbesondere flexible ferromagnetische Bänder vorgesehen sein. Besagter Magnetverschluss weist ähnliche Vorzüge in Bezug auf die Bedienbarkeit auf. Er kann ebenso gasdicht ausgeführt sein. Ebenso kann er technisch so ausgeführt sein, dass er auch unter den genannten erschwerten Bedingungen seine Gasdichtigkeit beibehält.

Alternativ zu obiger Ausführungsform kann vorgesehen sein, dass besagter gasdichter Verschluss in Form eines Siegel/Klebeverschlusses ausgeführt ist. Dabei werden zwei Folien in einer Siegelzone fest miteinander verschlossen. Mit einer Aufreißlasche kann die erste Folie leicht von der zweiten Folie gezogen werden; die in einer Mulde integrierte Klebschicht wird dabei freigelegt. Eine Trägerschicht aus Polyester, eine drucksensible Adhäsiv- und eine Siegelschicht mit integrierter Migrationsbarriere bilden die Komponenten der Unterfolie. Die Stärken liegen zwischen 200 - 500 µm. Besagter Siegel/Klebeverschluss ist beispielsweise aus der EP2067717 bekannt und weist ähnliche Vorzüge in Bezug auf die Bedienbarkeit auf. Er kann ebenso gasdicht ausgeführt sein Ebenso kann er technisch so ausgeführt sein, dass er auch unter den genannten erschwerten Bedingungen seine Gasdichtigkeit beibehält.

Alternativ zu obiger Ausführungsform kann vorgesehen sein, dass besagter gasdichter Verschluss in Form eines Klettverschlusses, ggf. mit innenliegender Dichtungslippe ausgeführt ist. Besagter Klettverschlusse weist ähnliche Vorzüge in Bezug auf die Bedienbarkeit auf. Er kann ebenso gasdicht ausgeführt sein. Ebenso kann er technisch so ausgeführt sein, dass er auch unter den genannten erschwerten Bedingungen seine Gasdichtigkeit beibehält.

Alternativ zu obiger Ausführungsform kann vorgesehen sein, dass besagter gasdichter Verschluss in Form eines Dichtungsgummis oder eines Schlauchgummis ausgelegt ist, ggf. mit einer Druckbeaufschlagung. Alternativ zu obiger Ausführungsform kann vorgesehen sein, dass besagter gasdichter Verschluss in Form eines Korkbandes ausgelegt ist, ggf. mit einer Druckbeaufschlagung.

Besagte Druckbeaufschlagung kann beispielsweise mittels eines Bügels oder des oben bereits erwähnten Klettverschlusses ausgeführt sein.

Alternativ zu obiger Ausführungsform kann vorgesehen sein, dass besagter gasdichter Verschluss in Form eines Klebeverschlusses ausgeführt ist, beispielsweise mit einem Low-Adhesive-Silikonkleber, der auf der Rahmen- oder der Fensterseite angebracht ist, bevorzugt in Form eines Films oder einer Beschichtung. Ebenso kann ein Acrylatkleber zum Einsatz kommen. Beide haben neben günstigen Dichtungs- und Klebeigenschaften dem Vorteil, dass sie physiologisch unbedenklich sind.

Bevorzugt ist vorgesehen, dass das Wundabdeckungselement mithilfe eines adhäsiven Materials an der Haut eines Patienten befestigbar ist. Hierbei kann jede Art von physiologisch akzeptablem Kleber verwendet werden, insbesondere alle medizinischen Kleber. Besonders bevorzugt ist das Material ausgewählt ist aus der Gruppe enthaltend
- Acrylatkleber
- Silikon
- Hydrokolloidkleber
- Zinkoxid-Kleber, und/oder
- Latexkleber

Hydrokolloidkleber bestehen in der Regel aus einem dünnen Polymerfilm, der auf einer selbstklebenden Masse aufgebracht ist. In der Trägersubstanz (z.B. synthetische Kautschukarten wie Polyisobutylen) befinden sich quellende Partikel, die je nach Hersteller etwas unterschiedlich sind. Oftmals sind Quellpartikel wie Carboxymethylcellulose oder Natriumcarboxymethycellulose enthalten. Sie sind ferner insbesondere nach Erwärmen gut modellierbar. Hydrokolloidkleber lassen sich sehr gut in flächigen Bahnen ausarbeiten und weisen insbesondere die Fähigkeit auf, Feuchtigkeit auszunehmen. Sie sind in pastöser Form lieferbar, aber auch in Platten- und Bahnenform.

Etwas ähnliches gilt für Silikonmaterialien. Bei Letzteren lässt sich insbesondere der Haftungsgrad an der Haut sehr gut einstellen, so dass trotz sicherer Haftung ein atraumatischer Verbandwechsle sichergestellt werden kann. Bevorzugt kann ein solcher Silikonkleber in Form eines ablösbaren Haftlaminats ausgebildet sein, das eine Strukturschicht umfasst, die auf der wundzugewandten Seite ein hydrophobes Gel, beispielsweise in Form eines Silikongels, und auf der Wundabgewandten Seite einen Haftkleber beispielsweise in Form eines Acrylat-Klebers trägt. Eine solche Schicht ist beispielsweise in der EP2001424B1 beschrieben.

Bevorzugt ist dabei vorgesehen, dass besagtes adhäsives Material in Form eines "Border-Dressings" als ein Kleberand ausgeführt ist, der peripher das Wundabdeckungselement umgibt.

Ebenso kann vorgesehen sein, dass besagtes adhäsives Material in Form einer Platte oder einer Bahn ausgeführt ist, an welcher das Wundabdeckungselement distal angeordnet ist. In dieser Ausführungsform kann beispielsweise vorgesehen sein, dass besagte Platte oder Bahn eine zentrale Öffnung aufweist, die über der Wunde zu liegen kommt. Besagte Platte oder Bahn ist dann wie ein Rahmen gestaltet. Alternativ kann vorgesehen sein, dass besagte Platte oder Bahn so ausgestaltet ist, dass entsprechend der Kontur der Wunde ein Fenster in die Platte oder Bahn geschnitten werden kann. Hierzu kann z.B. die Kontur der Wunde aufgezeichnet und dann entsprechend mit einer Schere ausgeschnitten werden. Ebenso kann eine Schablone verwendet werden, mit deren Hilfe die Wundkontur auf die Platte bzw. Bahn übertragen bzw. anhand der die Wundkontur an der Platte bzw. der Bahn ausgeschnitten werden kann.

Besagte Platte bzw. besagter Rahmen besteht beispielsweise aus einem wir hierin beschriebenen Hydrokolloidmaterial. Besagte Bahn besteht beispielsweise aus einer sogenannten Inzisionsfolie. Hierbei handelt es sich um eine selbstklebende Folie aus einem Polymermaterial.

Besagte Platte bzw. besagter Rahmen besteht alternativ aus einem Schaumstoffmaterial und/oder einem Abstandsgewirke. Dieses kann bevorzugt in eine gasdichte Hülle eingearbeitet sein. Hautseitig können dabei die oben erwähnten Kleber vorgesehen sein.

Erfindungsgemäß ist vorgesehen, dass die Vorrichtung ferner einen Wundexsudate absorbierenden Absorptionskörper aufweist.

Auf diese Weise kann sichergestellt werden, dass durch die Unterdrucktherapie geförderte Wundexsudate nicht vollumfänglich in einen externen Kanister überführt werden müssen, sondern mindestens teilweise im Wundraum verbleiben. Sie können durch einfaches Austauschen des Absorptionskörpers entfernt und, da im Absorptionskörper gebunden, leichter und hygienischer als ein exsudatgefüllter Kanister entsorgt werden.

Der hierdurch mögliche Verzicht auf einen externen Kanister hat weitere Vorteile; so kann die Vorrichtung so ausgestaltet werden, dass der Patient mobil bleibt (d.h. er kann das Bett verlassen und einem normalen Alltag nachgehen).

Erfindungsgemäß ist weiterhin vorgesehen, dass der Absorptionskörper mindestens eine superab sorbierende Sub stanzaufwei st.

Besonders bevorzugt ist vorgesehen, dass der Absorptionskörper ferner ein Vlies aufweisend Zellulosefasern aufweist.

Superabsorbierende Polymere (SAP) sind Kunststoffe, die in der Lage sind, ein Vielfaches ihres Eigengewichts - bis zum 1000-fachen - an Flüssigkeiten aufzusaugen. Chemisch handelt es sich dabei um ein Copolymer aus Acrylsäure (Propensäure, C₃H₄O₂) und Natriumacrylat (Natriumsalz der Acrylsäure, NaC₃H₃O₂), wobei das Verhältnis der beiden Monomere zueinander variieren kann. Zusätzlich wird ein so genannter Kernvernetzer (Core-Cross-Linker, CXL) der Monomerlösung zugesetzt, der die gebildeten langkettigen Polymermoleküle stellenweise untereinander durch chemische Brücken verbindet (sie "vernetzt"). Durch diese Brücken wird das Polymer wasserunlöslich. Beim Eindringen von Wasser oder wässrigen Salzlösungen in den Polymerpartikel quillt er auf und strafft auf molekularer Ebene dieses Netzwerk, so dass das Wasser ohne Hilfe nicht mehr entweichen kann. Die superabsorbierenden Polymere können in dem erfindungsgemäßen Wundpflegeartikel in Form eines Granulats, eines Pulvers, einer Schüttung, eines Presslings, eines Schaums, in Form von Fasern, eines Fasergewirkes, -geleges oder -vlieses und/oder einer Faserwatte vorliegen.

Bei modifizierter Cellulose handelt es sich bevorzugt um Derivate der Cellulose, bevorzugt Sulfoalkylierte Cellulose und deren Derivate, bevorzugt Celluloseethylsulfonate, Carboxyalkylierte Cellulose, bevorzugt Carboxymethylzellulose, Carboxyethylcellulose und/oder Carboxypropylcellulose, Komplexere Cellulosederivative, wie Sulphoethylcarboxymethylcellulose, Carboxymethylhydroxyethylcellulose, Hydroxy-propylmethylcellulose, und Amidierte Cellulosederivate, wie Carboxymethylcellulose-Amid oder Carboxypropylcellulose-Amid. Carboxymethylcellulose liegt insbesondere in Form von Natriumcarboxymethylcellulose vor und ist unter dem Namen "Hydrofaser" im Handel. In Hygiene- und Wundprodukten werden die Fasern in eine flächige Matrix überführt. Durch die Aufnahme von Flüssigkeit aus dem Wundexsudat werden die Fasern nach und nach in ein Gelkissen umgewandelt, das die Flüssigkeit hält und nicht wieder freigibt. Dabei sind die Fasern so aufgebaut, dass das Wundexsudat nur in vertikaler Richtung aufgenommen wird. Dies bedeutet, dass, solange die Kapazität reicht, das Exsudat nicht über den Wundrand fließt. Auf diese Weise kann eine Wundrandmazeration effektiv verhindert werden.

Bei besagten hydroaktiven Polymeren kann es sich auch um Alginate handeln. Alginate werden aus den Braunalgen gewonnen und zu einem faserigen Vlies verwoben Chemisch handelt es sich um Polysaccharide, und zwar Calcium- und/oder Natriumsalze der Alginsäuren. Alginate können bis zum 20fachen ihres Eigengewichtes an Flüssigkeit aufnehmen, dabei wird das Wundexsudat in die Hohlräume eingelagert. Die im Alginatgitter enthaltenen Ca2+ Ionen werden gegen die Na+ Ionen aus dem Exsudat ausgetauscht, bis der Sättigungsgrad an Na-Ionen im Alginat erreicht ist. Dabei kommt es zu einem Aufquellen der Wundauflage und zur Umwandlung der Alginatfaser in einen Gelkörper durch Aufquellen der Fasern.

Ebenso kann es sich bei besagten hydroaktiven Polymeren auch um Hydrogel-Nanopartikel aufweisend Hydroxy-Terminierte Methacrylatmonomere, wie 2-Hydroxyethylmethacrylat (HEMA) und/oder 2-Hydroxypropylmethacrylat (HPMA), die z.B. als Altrazeal vermarktet werden, handeln.

In einer weiteren bevorzugten Ausgestaltung ist vorgesehen, dass der Absorptionskörper einen Anteil von ≥ 40 Gew.-% an superabsorbierenden Polymeren aufweist. Besonders bevorzugt beträgt der Gewichtsanteil der superabsorbierenden Polymere ≥ 45, 50, 55, 60, 65 oder 70 Gew.-%.

Besonders bevorzugt ist vorgesehen, dass der Absorptionskörper ferner ein Vlies aufweisend Zellulosefasern aufweist.

Der Absorptionskörper kann bevorzugt einen im wesentlichen flachen Absorptionskörper aus Absorptionsmaterial aufweisen, der aus einem aufsaugenden Vlies mit darin verteilten superabsorbierenden Polymeren besteht. Diese können in Form eines Granulats, eines Pulvers, einer Schüttung, eines Presslings, eines Schaums, in Form von Fasern, eines Fasergewirkes, - geleges oder -vlieses und/oder einer Faserwatte vorliegen.

Dabei weist der Absorptionskörper mindestens ein Material auf, das ausgewählt ist aus der Gruppe enthaltend eine Matte, insbesondere aus einem Airlaid aus besagten Garnen oder Fasern aus superabsorbierenden Polymeren mit eingearbeiteten superabsorbierenden Polymeren, und/oder eine lose Füllung aus superabsorbierenden Polymeren. Besagte Airlaidmatte kann bevorzugt einen im wesentlichen flachen Materialabschnitt aus Absorptionsmaterial aufweisen, der z. B. aus einem aufsaugenden Vlies aus den genannten Fasern mit darin verteilten superabsorbierenden Polymeren besteht.

Dieser Absorptionskörper kann der absorbierenden Einlage entsprechen, die in einer Wundauflage der Anmelderin der vorliegenden Erfindung enthalten ist, wie sie beispielsweise in der WO03094813, der WO2007051599 und der WO0152780 offenbart ist und unter dem Handelsnamen "sorbion sachet" vertrieben wird.

Der Absorptionskörper kann in einer anderen Ausgestaltung ebenso einen Kern bilden, der - ggf. flockenartige - Fasern oder Garne aus superabsorbierenden Polymeren sowie superabsorbierenden Polymeren in Granulatform aufweist, wobei die Granulate an die Fasern bzw. Garne in mehreren Höhen angeklebt bzw. angeschweißt sind, und die Granulate über mehr als 50 % der gesamten Bauhöhe wenigstens eines Abschnitts des Kerns verteilt sind, wobei vermengte Bereiche von Granulat und Fasern vorliegen. Der Gewichtsanteil der superabsorbierenden Polymeren kann dabei bevorzugt im Bereich zwischen 10 - 25 Gew.-% liegen. Ähnliche Konstruktionen sind aus herkömmlichen Inkontinenzmaterialien bekannt und wie Hygienebinden für ihre polsternden Eigenschaften bekannt. Um besagten Kern herum kann eine Hülle angeordnet sein, die in Bereichen überlappend angeordnet ist, und der z.B. eine Klebenaht überdeckt bzw. Teil derselben ist.

Besonders bevorzugt weist der Absorptionskörper ein Vlies, bevorzugt ein Nonwoven oder Airlaid, auf, dass aus Superabsorbierenden Fasern ("SAF", bevorzugt Polyacrylate) besteht oder diese als Bestandteil enthält. Die Fasern können beispielsweise mit Fluff Pulp (Zellulose) oder mit Polyesterfasern gemischt sein. Alternativ oder Zusätzlich kann ein Schichtaufbau vorgesehen sein.

| Typ | 1 | 2 | 3 | 4 | 5 | 6 |
|---|---|---|---|---|---|---|
| Aufbau 1 | Schichtaufbau: Thermogebondetes Airlaid mit laminiertem Nonwoven | 40% Polyester Kurzschnittfaser; 60% SAF | Bicomponentenfaser aus SAF und einem Thermoplasten | Schichtaufbau: Thermogebondetes Airlaid mit laminiertem Nonwoven | 25% Polyester; 75% SAF | 40% Polyester Kurzschnittfaser; 60% SAF |
| Aufbau 2 | Bicomponentenfaser aus SAF und einem Thermoplasten + Fluff-Pulp | Nadelfilz | Kardiertes, thermogebondetes Nonwoven | Bicomponentenfaser aus SAF und einem Thermoplasten + Fluff-Pulp | Nadelfilz | Nadelfilz |
| Typ der SAF Faser | 101/6/10 | 102/52/10 | 102/52/10 | 101/6/10 | | |
| Gewicht (g/m²) | 560 | 540 | 1000 | 350 | 150 | 380 |
| Dicke (mm) | 6 | 5,4 | 20 | 3,5 | 2,4 | 3,8 |
| Aufnahmekapazität | 31,2 l Wasser/m² | > 20 g Wasser/g | > 16 g Wasser/g bzw. 16000 g Wasser/m² | 19,5 l Wasser/m² | > 25g 0.9 % Kochsalz/g | > 17 g Wasser/g bzw. 6400 g/m² |
| Aufnahmekapazität unter Druck (ml 0.9% Kochsalz/m² bei 0,2 psi Druck) | 16 | | | 16 | | |
| Gesamtgehalt an Superabsorbierenden Polymeren (% w/w) | 18 | 40 | 50 | 18 | 75 | 60 |
| Zugfestigkeit (N/5cm) | | | | | 16±13 | 16±13 |
| Dehnbarkeit (%) | | | | | 60±18 | 60±18 |

Der Absorptionskörper kann in einer anderen Ausgestaltung ebenso mindestens eine flache Lagen aufweisend Fasern oder Garne aus superabsorbierenden Polymeren enthalten, an welche superabsorbierende Polymere in Granulatform geklebt sind. Dadurch ergibt sich in einer bevorzugten Ausgestaltung ein Aufbau des Körpers, der wenigstens drei Schichten aufweist, wobei zwei Deckschichten eine Schicht aufweisend superabsorbierende Polymere umgeben.

Dabei liegen in der Ebene keine Vermengungen von Fasern und superabsorbierenden Polymeren vor; sondern lediglich fixierte Benachbarungen beider Materialien. Die ggf. vorgesehenen mehreren Lagen können dabei in einer bevorzugten Ausgestaltung auch durch Walzen, Pressen, Kalandrieren oder ähnliche Verfahren physisch miteinander verdichtet sein.

| Typ | 1 | 2 | 3 | 4 | 5 | 6 | 7 | 8 |
|---|---|---|---|---|---|---|---|---|
| Gewicht (g/m²) | 450 | 300 | 150 | 50 | 100 | 120 | 140 | 440 |
| Dicke (mm) | 1,3 | 1,2 | 0,9 | 0,7 | 0,7 | 0,76 | 1 | 1,2 |
| Flüssigkeitsretention (g/g) | 28 | 33 | 28 | 15 | 25 | 28 | 11,5 | 38 |
| Zugfestigkeit (N/5cm) | 25 | 55 | 20 | 20 | 20 | 20 | 15 | 20 |
| Aufnahmekapazität (g/g) | 45 | 20 | 50 | 20 | 40 | 50 | 28 | 55 |

Überdies kann der Körper sich wiederholende Musterungen oder Maserungen aufweisen, wie z.B. ein Karomuster, ein Stanzmuster oder dergleichen.

Besonders bevorzugt weist besagter Absorptionskörper ein Flächenmaß von 5 × 5, 5 × 10, 5 × 20, 10 × 10, 10 × 15, 10 × 20, 15 × 15, 20 × 20 oder 20 × 40 cm auf.

Besonders bevorzugt ist ferner vorgesehen, dass besagter Absorptionskörper neben einer Lage aufweisend superabsorbierende Polymere mindestens eine zweite flankierende Lage aufweist, die weniger oder keine superabsorbierenden Polymere aufweist und flächenmäßig über erstgenannte hinausragt. Auf diese Weise wird sichergestellt, dass die Lage aufweisend superabsorbierende Polymere entsprechend der Flüssigkeitsaufnahme an Volumen gewinnen kann, ohne dass die Volumenzunahme nach außen hin erkennbar ist, weil letztere durch die zweite Schicht kaschiert wird.

Als Schaumstoffe kommen sowohl geschlossen- als auch offenporige Schaumstoffe in Frage. Diese sind bevorzugt ebenfalls als flächige Lage ausgestaltet und weisen einerseits flüssigkeitsaufnehmende Eigenschaften und andererseits polsternde Eigenschaften auf. Außerdem weisen sie hohe Rückstellkräfte auf. Bevorzugt werden insbesondere sogenannte Kaltschäume verwendet.

Alternativ oder in Ergänzung zu besagtem Schaum können auch sogenannte "nanofiber matrices" verwendet werden, wie sie beispielsweise von der Firma SNS Nano Fiber hergestellt werden.

Ferner können besagte Schäume auch mit Superabsorbern versetzt sein, wie dies beispielsweise im Produkt Allevyn Plus der Firma Smith & Nephew erfolgt ist.

Als ein Aspekt, der nicht erfindungsgemäß ist und lediglich der Veranschaulichung dient, ist weiterhin vorgesehen, dass die Vorrichtung ferner ein wundzugewandtes flüssigkeitsdurchlässiges Wunddistanzgitter aufweist.

Bevorzugt handelt es sich bei einem solchen Wunddistanzgitter um ein Kunststoffgitter (bevorzugt beispielsweise aufweisend ein Silikonmaterial oder ein Nylonmaterial), einen gelochten Schaumstoff, ein Abstandsgewirke und/oder eine perforierte Folie.

Bevorzugt ist ein dreidimensionales Wunddistanzgitter vorgesehen, wie es beispielsweise unter dem Markennamen "sorbion plus" am Markt und in der EP2004116A1 beschrieben ist.

Dabei kann das Wunddistanzgitter einerseits wundseitig vorgesehen sein, und sowohl lose innerhalb des Rahmen anordenbar sein als auch am Rahmen fixiert sein.

Ein solches Wunddistanzgitter verhindert die Eingranulierung und ermöglicht damit einen atraumatischen Verbandwechsel, hat ferner eine Biofilmauflösende Wirkung und außerdem einen Ventileffekt, dergestalt, dass ein Rückfluss von Exsudat reduziert wird.

Die Anmelderin konnte zeigen, dass diese Eigenschaften, die aus "passiven" Wundauflagen bekannt sind, auch bei den hier beschriebenen "aktiven" vakuumunterstützten Wundauflagen hervorragende Vorteile bieten.

Erfindungsgemäß ist ein deckelseitiges Wunddistanzgitter deckelseitig vorgesehen. Hierbei kann es ein Gelblocking verhindern, dass durch einen etwaig dazwischen liegenden Absorptionskörper aufweisend superabsorbierende Polymere verursacht wird, wenn dieser durch Flüssigkeitsaufnahme ein Gel ausbildet, das ansonsten den Absaugprozess von Flüssigkeiten behindern kann.

Die erwähnte Anschlussvorrichtung zum Ansaugen von fluiden Medien kann grundsätzlich so ausgestaltet sein, dass sie das Absaugen von Flüssigkeiten und/oder Gasen ermöglicht. Hierzu kann sie z.B. in Form einer Kupplung ausgestaltet sein (etwa nach dem Luer-Lock-System), die das Anschließen eines Schlauches und/oder einer Pumpe ermöglicht.

Ferner kann das Produkt ein zusätzliches Ventil, eine Druckminderer oder gar ein weiteres öffenbares Fenster aufweisen. Hiermit kann sichergestellt werden, dass die Vorrichtung auch bei einem zu starken Unterdruck verwendet werden kann, beispielsweise mit einem in Krankenhäuser oft vorgesehen Vakuumanschluss in der Wand. Besagtes Ventil, Druckminderer oder öffenbares Fenster kann dann geöffnet werden, um den Unterdruck zu mindern.

Bevorzugt ist weiterhin vorgesehen, dass im Bereich der Anschlussvorrichtung zum Ansaugen von fluiden Medien eine Barriere angeordnet ist, die keine Flüssigkeiten passieren lässt.

Auf diese Weise wird sichergestellt, dass keine Flüssigkeit in die Pumpe gelangt. Letztere verbleibt innerhalb der Wundabdeckung und wird von Wundexsudate aufsaugenden Absorptionskörper aufgenommen.

Besagte Barriere weist bevorzugt eine semipermeable Membrane auf, beispielsweise aus einem Material wie Goretex, etc..

Bevorzugt kann jeweils eine Anschlussvorrichtung zum Ansaugen von flüssigen Medien und eine Anschlussvorrichtung zum Ansaugen von gasförmigen Medien vorgesehen sein.

Bevorzugt ist weiterhin vorgesehen, dass die Vorrichtung ferner eine Einrichtung zur Erzeugung von atmosphärischem Unterdruck aufweist.

Besagte Einrichtung zur Erzeugung von atmosphärischem Unterdruck ist bevorzugt ausgewählt aus der Gruppe enthaltend
a) Elektrisch betriebene Vakuumpumpe,
b) Handbetriebene Unterdruckquelle, und/oder
c) Evakuiertes Vakuumgefäß.

Die besagte Vakuumpumpe kann eine solitäre Pumpe sein, sie kann jedoch auch Bestandteil eines zentralisierten Saugsystems sein, wie es in Kliniken häufig zum Einsatz kommt. Dabei werden in den Patientenzimmern wandinstallierte Vakuumanschlüsse bereitgestellt, an welche die erfindungsgemäßen Drainagevorrichtungen zur Wundbehandlung angeschlossen werden können. In diesem Fall kann besagte Vakuumpumpe eine Vielzahl von erfindungsgemäßen Drainagevorrichtungen zur Wundbehandlung unter Unterdruck setzen.

Bevorzugt handelt es sich um eine Mikropumpe, deren Ausmaße und/oder deren Gewicht so beschaffen ist, dass sie ohne weiteres an einem Wundabdeckungselement der genannten Art angebracht werden kann, ohne dass dies dem Patienten lästig fällt oder ihn in irgendeiner Weise stört.

Diese Pumpe kann beispielsweise vom Typ Piezo- oder Membranpumpe sein. Besonders bevorzugt sind Piezopumpen, also Pumpen, bei denen die Pumpleistung durch ein piezoelektrisches Element bewerkstelligt wird. Diese Pumpen weisen eine ausreichend hohe Pumpleistung bei geringen Ausmaßen, geringem Betriebsgeräusch und niedriger Energieaufnahme auf. Ebenso kann es sich um eine in Mikrosystemtechnik gefertigte Treibmittelvakuumpumpe handeln. Geeignete Pumpen dieses Typs werden beispielsweise von Schwarzer Precision, KNF oder Bartels Mikrotechnik hergestellt.

Bevorzugt ist eine solche Pumpe ferner mit einem Rückschlagventil ausgestattet. Auf diese Weise kann die Pumpe im Intervallmodus betrieben werden oder lediglich zum initialen Aufbau des Unterdrucks bzw. zum Erhalt des Unterdrucks eingeschaltet werden, ohne dass in den Betriebspausen Leckagen entstehen, die den aufgebauten Unterdruck wieder abbauen.

Bevorzugt ist die besagte Pumpe imstande, Unterdrücke von -60 bis -200 mm Hg zu ziehen. Ganz besonders bevorzugt ist vorgesehen, dass die Pumpe Unterdrücke von -60, -70, -80, -85, -90, -100, -110, -120, -130, -140, -150, -160, -170, -180, -190 oder - 200 mm Hg zieht.

Bevorzugt ist die Pumpe so gewählt bzw. ausgestaltet, dass sie in der Lage ist, Flüssigkeiten zu befördern.

Bevorzugt überschreitet das Betriebsgeräusch der Pumpe einen Schalldruckpegel von 65 dB nicht. Besonders bevorzugt wird ein Schalldruckpegel von 63 dB, 60 dB, 58 dB, 55 dB, 53 dB, 50 dB, 48 dB, 45 dB, 42 dB, 40 dB, 38 dB, 35 dB, 32 dB, 30 dB, 28 dB, 25 dB 22 dB, oder sogar 20 dB nicht überschritten.

Bevorzugt weist die Pumpe eine Förderrate zwischen 0,5 ml min⁻¹ und 100 ml min⁻¹ auf. Bevorzugt sind Förderraten zwischen 2 ml min⁻¹ und 50 ml min⁻¹ . Besonders bevorzugt sind Förderraten zwischen 10 ml min⁻¹ und 20 ml min⁻¹.

Besagtes evakuiertes Gefäß kann ähnlich der bekannten Redonflasche an die erfindungsgemäße Vorrichtung zur Wundbehandlung angeschlossen werden und diese so unter Unterdruck setzen. Besagtes evakuiertes Gefäß weist eine flüssigkeitsabsorbierende Polymere enthaltende Einlage auf, bevorzugt in Form einer Wandauskleidung.

Besonders bevorzugt kann besagtes evakuiertes Gefäß in Form einer Patrone vorgesehen sein, die in eine Halterung eingelegt wird, die bereits mit der erfindungsgemäßen Drainagevorrichtung zur Wundbehandlung verbunden ist. Wenn die Patrone voll ist, wird sie entnommen und entsorgt, und eine neue evakuierte Patrone kann in die Halterung eingelegt werden.

Besonders vorteilhaft sind die vorgenannten Ausgestaltungen, weil durch Verzicht auf eine eigene Pumpe und stattdessen Verwendung eines evakuierten Gefäßes die Vorrichtung mobil und netzunabhängig wird, so dass der Patient selbst mobil wird. Darüber hinaus kann so eine kleinere Bauweise erzielt werden, die es dem Patienten ermöglicht, die Vorrichtung diskret zu verbergen. Vorteilhaft ist hierzu insbesondere eine anatomisch angepasste Ausgestaltung des besagten evakuierten Gefäßes bzw. der erwähnten Halterung, was ein unauffälliges Tragen derselben beispielsweise am Bein ermöglicht.

Ferner macht eine solche Vorrichtung keinerlei Betriebsgeräusche und ist sehr leicht zu bedienen.

Ähnliches gilt für die erwähnte Handbetriebene Unterdruckquelle. Hier kann es sich im einfachsten Falle um eine Plastikspritze mit ausreichend hohem Volumen handeln. Andere Möglichkeiten liegen in einer einem Gummiball ähnlichen Pumpe, einem Faltenbalg etc..

Bevorzugt ist weiterhin vorgesehen, dass die Einrichtung zur Erzeugung von atmosphärischem Unterdruck unmittelbar an der Wundpflegevorrichtung angeordnet ist.

Dies kann beispielsweise dergestalt erfolgen, dass besagte Einrichtung unmittelbar am Wundabdeckungselement angeordnet ist. Auf diese Weise ist ein gesonderter Vakuumschlauch, der fertigungstechnische Probleme (ausreichend hohe Steifheit, um nicht unter Vakuum zu kollabieren) sowie hygienische Probleme (Gefahr der Kontamination) aufweist, verzichtbar, bzw. kann sehr kurz gehalten werden.

Bevorzugt ist weiterhin vorgesehen, dass zwischen der Einrichtung zur Erzeugung von atmosphärischem Unterdruck und dem Wundabdeckungselement bzw. dem Wundexsudate aufsaugenden Absorptionskörper eine Kupplung, ein Absperrventil und/oder ein Dreiwegehahn angeordnet ist.

Mittels dieser Vorrichtung kann gewährleistet werden, dass a) die Einrichtung zur Erzeugung von atmosphärischem Unterdruck vom Rest der Vorrichtung abgekoppelt werden kann, b) ein einmal gezogener Unterdruck möglichst lange gehalten wird und/oder c) zwecks Schonung der Batterie oder bei entladender Batterie über eine externe Pumpe oder ein externes Vakuumgefäß initial oder erneut ein Unterdruck angelegt werden kann.

Besagte Externe Pumpe bzw. besagtes externes Vakuumgefäß kann beispielsweise in der Klinik oder beim Patienten zu Hause stehen, oder aber in mobiler Form ausgebildet sein (beispielsweise als in Form eines Koffers, integriert in die Kleidung des Patienten oder über einen Gurt am Patienten befestigt). So wird sichergestellt, dass die Vorrichtung an der Wunde klein und unauffällig bleibt - da ihre Aufgabe vor allem die ist, das Vakuum aufrecht zu erhalten, bei Bedarf jedoch ausreichend Pumpkapazität zur Verfügung steht, um eine effiziente Therapie zu gewährleisten.

Bevorzugt ist weiterhin vorgesehen, dass der Absorptionskörper mit einer flüssigkeitspermeablen Hülle umgeben ist. Bevorzugt weist die Vorrichtung ferner einen Abstandskörper auf. Dieser kann bevorzugt zwischen Wunde und Wundabdeckungselement platziert werden, ebenso aber auch - wenn vorhanden - zwischen Absorptionskörper und Wundabdeckungselement bzw. zwischen Wunde und Absorptionskörper platziert werden. Ein solcher Abstandskörper gewährleistet, dass die Wundpflegevorrichtung bei Anlegen eines Unterdrucks nicht vollständig kollabiert, was dazu führen würde, dass gase oder Flüssigkeiten nicht weiter abgesaugt werden könnten.

Bevorzugt ist weiterhin vorgesehen, dass das die Vorrichtung ferner eine Lage aufweisend ein Schwermetall, bevorzugt Kupfer oder Silber, oder ein Salz desselben, aufweist. Gerade in Bezug auf eine verlängerte Verweildauer auf der Wunde kann eine solche Lage sehr hilfreich sein, da sie das Bakterienwachstum bremst.

Bevorzugt ist weiterhin vorgesehen, dass das Wundabdeckungselement flüssigkeits- und/oder gasundurchlässig ist. Auf diese Weise wird das Anlegen eines Vakuum ermöglicht, und es wird verhindert, dass Flüssigkeit, die u.U. kontaminiert sein kann, austritt.

Bevorzugt ist besagtes Wundabdeckungselement elastisch ausgebildet. Hierzu kann es beispielsweise aus ein Polypropylen-, Polyethylen-, Latex-, Silikon- oder Kautschukmaterial aufweisen. Ferner kann es auch auf der Innenseite mit einem Schaummaterial versehen sein.

Bevorzugt ist weiterhin vorgesehen, dass das Wundabdeckungselement wasserdampfdurchlässig ist. Bevorzugt ist das Wundabdeckungselement ferner transparent ausgebildet.

Bevorzugt ist weiterhin vorgesehen, dass die Einrichtung zur Erzeugung von atmosphärischem Unterdruck in ihrer Pumprichtung reversibel ist. Auf diese Weise lässt sich die Einrichtung auch als steuerbare Dosierpumpe für Medikamente, Spüllösungen etc. einsetzen.

Bevorzugt ist weiterhin vorgesehen, dass besagtes öffenbares Fenster eine nichtelastische Rückwand aufweist. Mit dem Begriff "Rückwand" ist der Deckel an sich gemeint, wie beispielsweise in Bezugszeichen 23 gezeigt. In dieser Ausführungsform kann beispielsweise die diskutierte Pumpe direkt auf der Rückwand des öffenbaren Fensters angeordnet sein.

Bevorzugt ist weiterhin vorgesehen, dass besagtes öffenbares Fenster eine elastische Rückwand aufweist. In dieser Ausführungsform kann beispielsweise vorgesehen sein, dass die Rückwand der durch Exsudataufnahme bedingten Volumenzunahme eines darunter angeordneten Absorptionskörpers nachgibt. Ebenso kann damit gewährleistet werden, dass das Fenster den Bewegungen des Patienten folgt und so die Dichtheit gewährleistet bleibt.

Bevorzugt weist diese elastische Rückwand ein Silikonmaterial auf bzw. besteht aus einem solchen. Besonders bevorzugt kann beispielsweise vorgesehen sein, dass die Unterseite der Rückwand - also die Patientenzugewandte Seite - mit einem Silikon- und/oder einem Schaummaterial beschichtet oder mit einem solchen Material unterlegt ist.

Bevorzugt ist weiterhin vorgesehen, dass besagtes öffenbares Fenster eine Rückwand aus einem plastisch verformbaren Material aufweist.

In dieser Ausführungsform kann beispielsweise vorgesehen sein, dass die Rückwand aus einer thermoplastisch verformbaren Tiefziehfolie besteht. Auf diese Weise kann ein Reservoir vorgesehen sein, innerhalb welchem beispielsweise ein Reserve-Absorptionskörper vorgesehen sein kann.

Bevorzugt ist weiterhin die Verwendung einer Wundpflegevorrichtung zur Behandlung von Wunden aufweisend Weichteildefekte, infizierten Wunden nach chirurgischem Debridement, Lymphfisteln, sternalen Wundinfektionen, Thoraxwandfenstern, Dekubitus, Ulcus cruris, chronischen Wundheilungsstörungen, Strahlenulkus, Abdominellem Kompartmentsyndrom, septischem Abdomen, enteraler Fisteln und/oder Wunden, die durch ein oder mehrere Ödeme hervorgerufen werden, zur Fixierung von Hauttransplantaten, zur Wundkonditionierung und/oder zur postoperativen Versorgung von Nähten und Inzisionen vorgesehen.

Ferner ist weiterhin die Verwendung einer Wundpflegevorrichtung gemäß einem der vorherigen Ansprüche in einem Wundkompressionssystem vorgesehen.

### Abbildungen

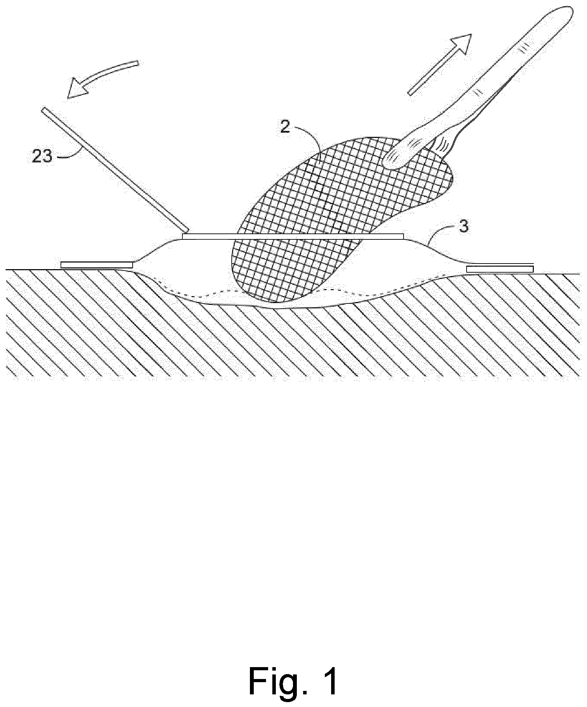

Die Figuren 1 bis 22 zeigen Wundpflegevorrichtungen, welche nicht Teil der Erfindung sind.

Fig. 1 zeigt das generelle Prinzip einer Wundpflegevorrichtung zur Behandlung von Wunden mittels atmosphärischem Unterdruck im Wundbereich, aufweisend ein Wundabdeckungselement, 3 das an der Haut eines Patienten befestigbar ist, sowie eine nicht dargestellte Anschlussvorrichtung zum Ansaugen von fluiden Medien. Das Wundabdeckungselement weist ein öffenbares Fenster 23 auf, das mittels eines nicht dargestellten gasdichten Verschlusses an dem Wundabdeckungselement angeordnet ist. Nach Öffnen des Fensters 23 kann eine Wundauflage 2 einfach entnommen, entsorgt und gegen eine andere Wundauflage ausgetauscht werden.

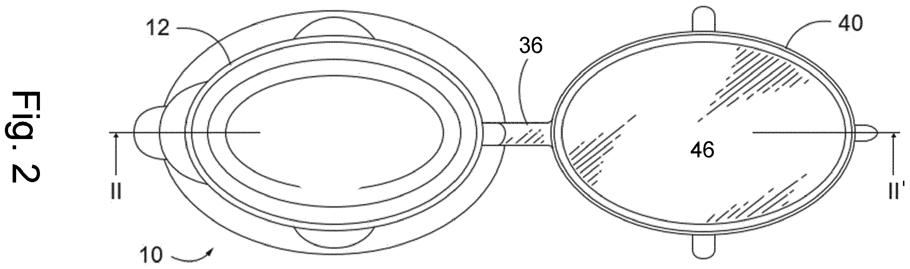

Fig. 2 zeigt ein öffenbares Fenster 10 aufweisend einen Rahmen 12, eine Verbindungslasche 36 zwischen Rahmen 12 und Fensterdeckel 44, sowie einen gasdichten Verschluss in Form eines Leistenverschlusses 18, 40.

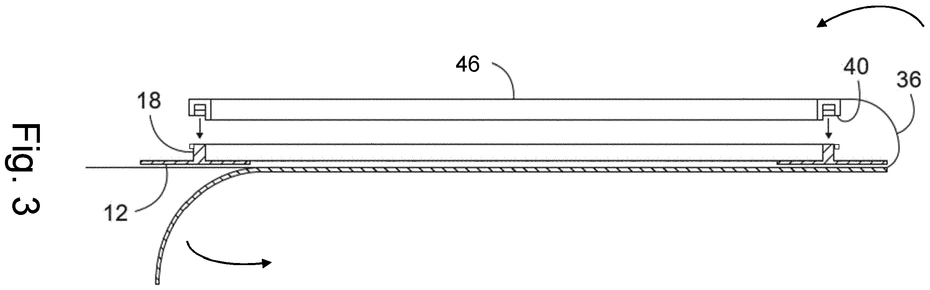

In Fig. 3 ist besagter Leistenverschluß 18, 40 im Querschnitt im Detail gezeigt.

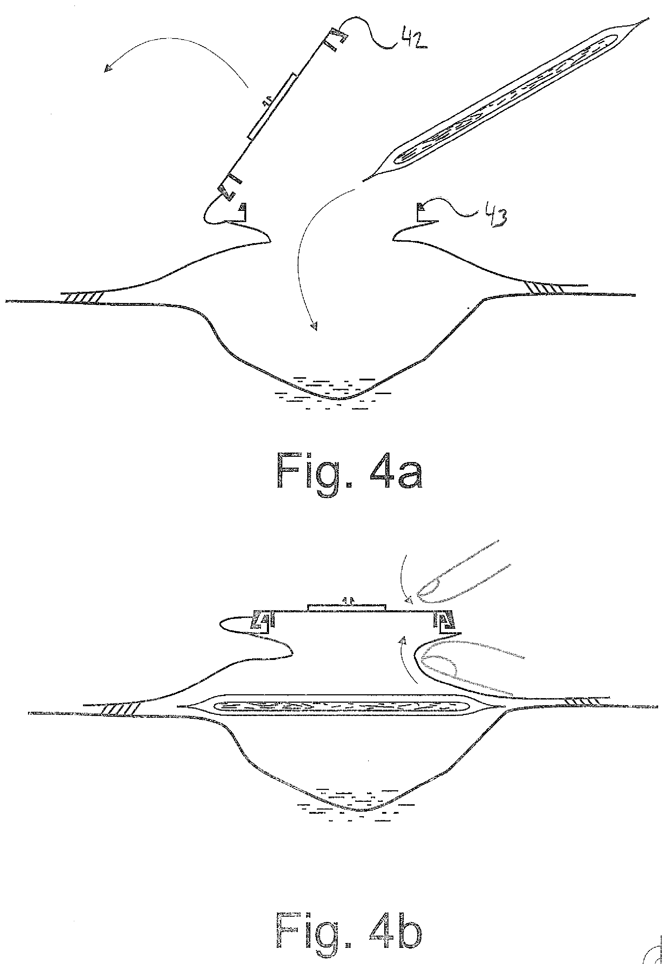

Fig. 4 zeigt ebenfalls ein öffenbares Fenster mit einem Leistenverschluss 42, 43. Besagter Leistenverschluss ist mit einer Hintergriffsmöglichkeit versehen, die dafür sorgt, dass der Leistenverschluss eine geringe Kraft beim Schließen bzw. Öffnen erfordert. Auf diese Weise kann das Öffnen bzw. Schließen ohne Schmerzeinwirkungen ausgeübt werden. Ferner weist der Verschluss eine geringe Bauhöhe und ein geringes Gewicht auf.

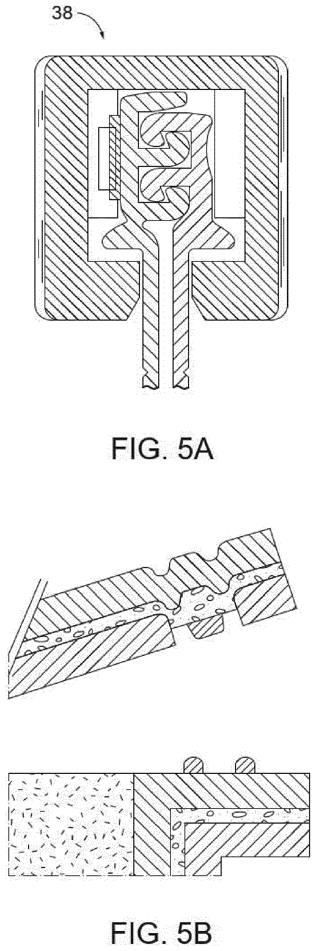

Fig. 5a zeigt eine andere Form eines erfindungsgemäß verwendbaren Leistenverschlusses in Form eines Gleiters 38, der ähnlich wie bei einem Reißverschluss entlang der zu verschließenden Lippen gleitet und sie in die Schließposition drängt.

Fig. 5b zeigt einen gasdichten Verschluss in Form eines Siegel/Klebeverschlusses.

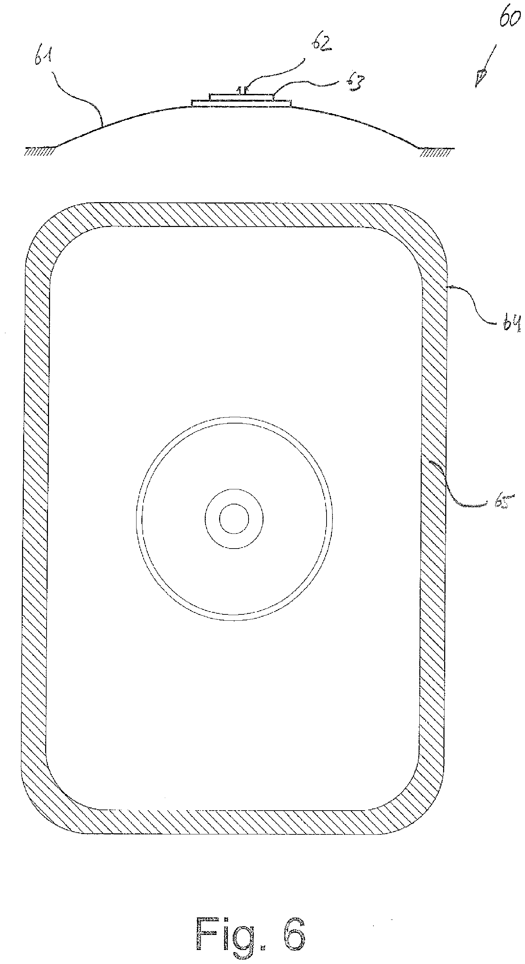

Fig. 6 zeigt eine Wundpflegevorrichtung 60 zur Behandlung von Wunden mittels atmosphärischem Unterdruck im Wundbereich, aufweisend ein Wundabdeckungselement 61, das an der Haut eines Patienten befestigbar ist sowie eine Anschlussvorrichtung 62 zum Ansaugen von fluiden Medien. Das Wundabdeckungselement weist ein öffenbares Fenster 63 auf, das mittels eines gasdichten Verschlusses an dem Wundabdeckungselement angeordnet ist.

Ferner weist das Wundabdeckungselement einen umlaufenden Rand 64 aufweisend ein adhäsives Material 65 auf. Auf diese Weise ist ein sogenanntes "Border-Dressing" verwirklicht.

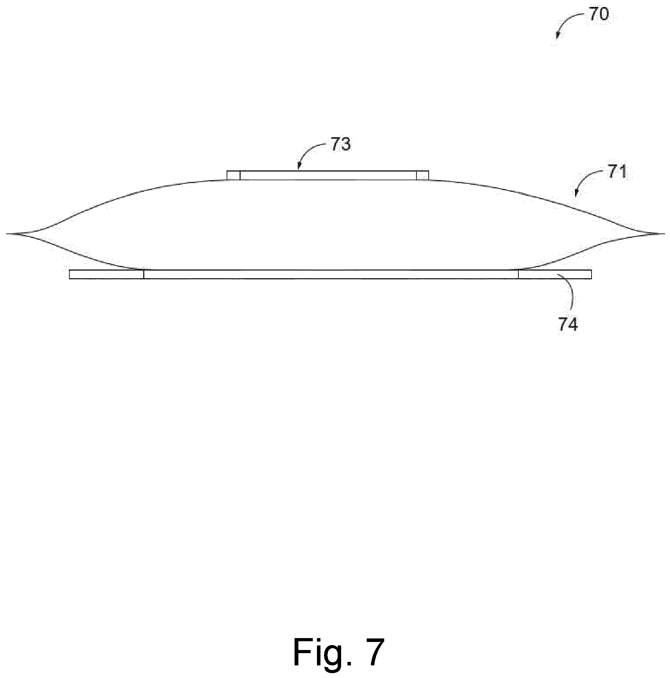

Fig. 7 zeigt eine Wundpflegevorrichtung 70 zur Behandlung von Wunden mittels atmosphärischem Unterdruck im Wundbereich, aufweisend ein Wundabdeckungselement 71, das an der Haut eines Patienten befestigbar ist sowie eine nicht dargestellte Anschlussvorrichtung zum Ansaugen von fluiden Medien. Das Wundabdeckungselement weist ein öffenbares Fenster 73 auf, das mittels eines gasdichten Verschlusses an dem Wundabdeckungselement angeordnet ist.

Die Vorrichtung weist ferner eine Platte oder einen Rahmen 74 auf, an welcher das Wundabdeckungselement distal angeordnet ist. In dieser Ausführungsform kann beispielsweise vorgesehen sein, dass besagte Platte oder Bahn eine zentrale Öffnung aufweist, die über der Wunde zu liegen kommt. Besagte Platte oder Bahn ist dann wie ein Rahmen gestaltet. Alternativ kann vorgesehen sein, dass besagte Platte oder Bahn so ausgestaltet ist, dass entsprechend der Kontur der Wunde ein Fenster in die Platte oder Bahn geschnitten werden kann. Besagte Platte besteht beispielsweise aus einem wir hierin beschriebenen Hydrokolloidmaterial. Besagte Bahn besteht beispielsweise aus einer sogenannten Inzisionsfolie. Hierbei handelt es sich um eine selbstklebende Folie aus einem Polymermaterial.

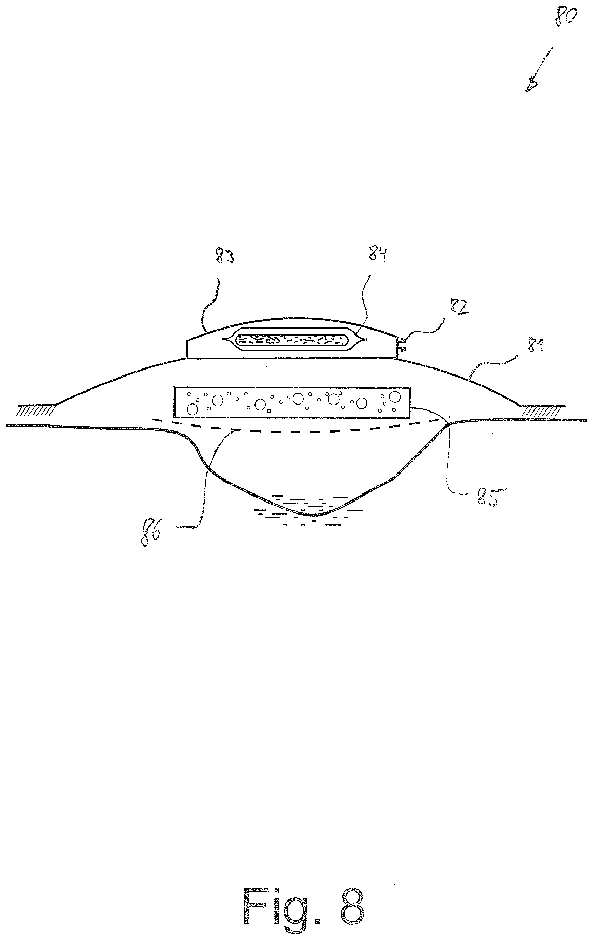

Fig. 8 zeigt eine Wundpflegevorrichtung 80 zur Behandlung von Wunden mittels atmosphärischem Unterdruck im Wundbereich, aufweisend ein Wundabdeckungselement 81, das an der Haut eines Patienten befestigbar ist sowie eine Anschlussvorrichtung 82 zum Ansaugen von fluiden Medien. Das Wundabdeckungselement weist ein öffenbares Fenster 83 auf, das mittels eines gasdichten Verschlusses an dem Wundabdeckungselement angeordnet ist.

Das öffenbare Fenster ist mit einer Druckresistenten Wandung ausgestattet und bietet Raum für einen Absorptionskörper 84 aufweisend Superabsorbierende Polymere. Durch die Druckresistenz kollabiert das öffenbare Fenster nicht, sodass der Absorptionskörper seine volle Aufnahmekapazität ausschöpfen kann.

Ferner ist wundseitig ein Durchlaufkörper 85 aus einem Schaumstoffmaterial sowie ein dreidimensionales Wunddistanzgitter 86 vorgesehen.

Fig. 9 zeigt eine Wundpflegevorrichtung 90 zur Behandlung von Wunden mittels atmosphärischem Unterdruck im Wundbereich, aufweisend ein Wundabdeckungselement 91, das an der Haut eines Patienten befestigbar ist, sowie eine Anschlussvorrichtung 92 zum Ansaugen von fluiden Medien.

Das Wundabdeckungselement weist ein öffenbares Fenster 93 auf, das mittels eines gasdichten Leistenverschlusses 94 an dem Wundabdeckungselement angeordnet ist. Der gezeugte Leistenverschluss ist auch unter den Pseudonymen "Ziplock", "Minigrip" oder Gleitverschluss bekannt. Er weist eine geringe Bauhöhe und ein geringes Gewicht auf und erfordert überdies eine geringe Kraft beim Schließen bzw. Öffnen.

Unterhalb des öffenbares Fensters 93 ist ein Fach 98 mit einer druckresistenten Wandung vorgesehen, dass Raum für einen Absorptionskörper 95 aufweisend Superabsorbierende Polymere bietet. Besagtes Fach ist zur Wundseite hin flüssigkeitsdurchlässig ausgebildet. Durch die Druckresistenz kollabiert das Fach nicht, sodass der Absorptionskörper seine volle Aufnahmekapazität ausschöpfen kann. Ist diese erreicht, kann das gesamte Fenster mitsamt dem Fach und dem darin enthaltenen Absorptionskörper entsorgt und gegen ein neues ausgetauscht werden.

Ferner ist wundseitig ein Durchlaufkörper 96 aus einem Schaumstoffmaterial sowie ein dreidimensionales Wunddistanzgitter 97 vorgesehen. Der Durchlaufkörper ist - anders als das Fach 98 - nicht druckresistent untergebracht und verringert daher bei Anlegen eines Drucks sein Volumen.

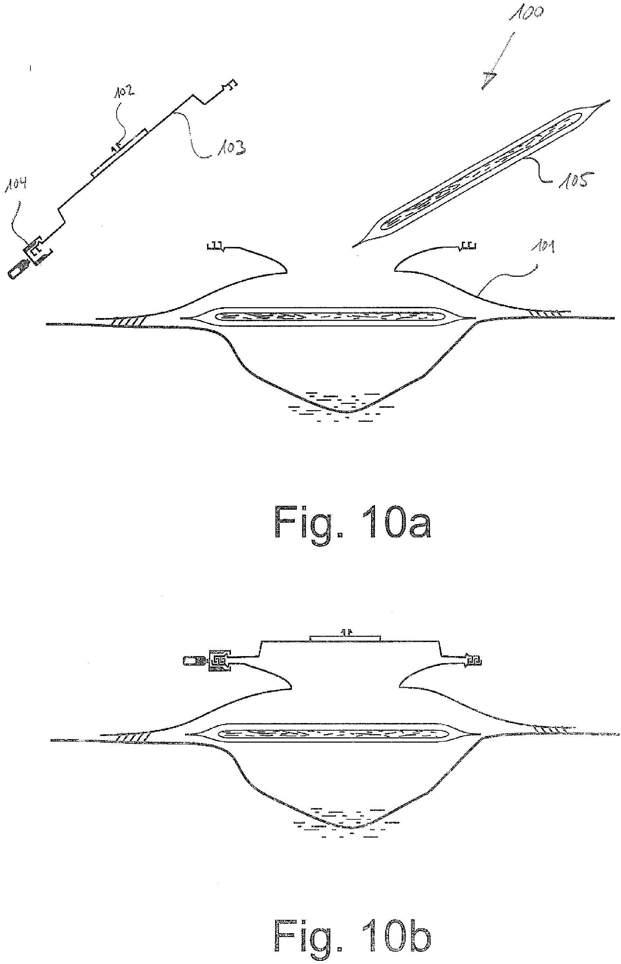

Fig. 10 zeigt eine Wundpflegevorrichtung 100 zur Behandlung von Wunden mittels atmosphärischem Unterdruck im Wundbereich, aufweisend ein Wundabdeckungselement 101, das an der Haut eines Patienten befestigbar ist, sowie eine Anschlussvorrichtung 102 zum Ansaugen von fluiden Medien.

Das Wundabdeckungselement weist ein öffenbares Fenster 103 auf, das mittels eines gasdichten Leistenverschlusses 104 an dem Wundabdeckungselement angeordnet ist. Der gezeigte Leistenverschluss ist auch unter den Pseudonymen "Ziplock", "Minigrip" oder Gleitverschluss bekannt. Er weist eine geringe Bauhöhe und ein geringes Gewicht auf und erfordert überdies eine geringe Kraft beim Schließen bzw. Öffnen.

Ferner ist ein Absorptionskörper 105 vorgesehen, der nach Öffnen des Fensters entnommen oder eingeführt werden kann.

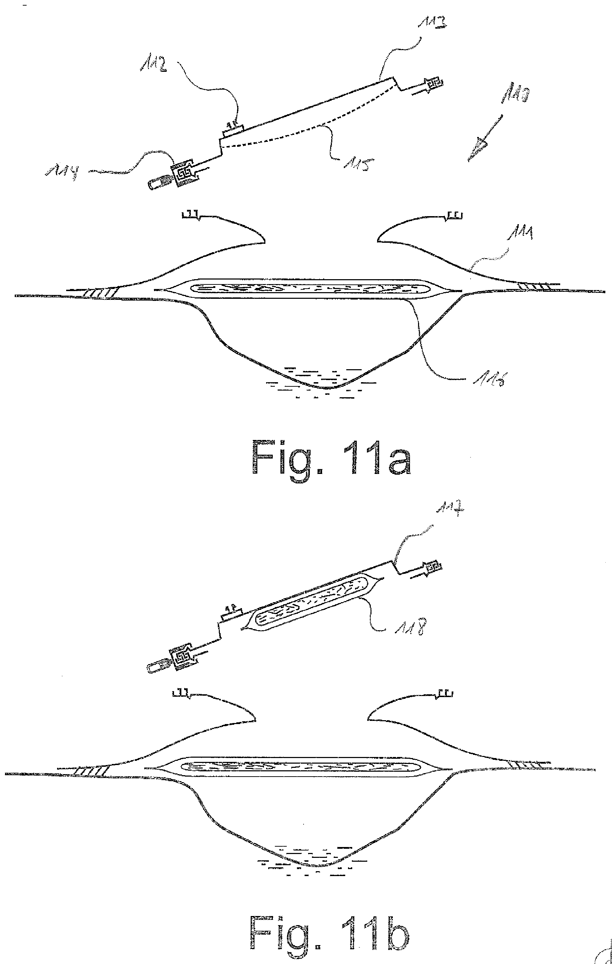

Fig. 11a zeigt eine Wundpflegevorrichtung 110 zur Behandlung von Wunden mittels atmosphärischem Unterdruck im Wundbereich, aufweisend ein Wundabdeckungselement 111, das an der Haut eines Patienten befestigbar ist, sowie eine Anschlussvorrichtung 112 zum Ansaugen von fluiden Medien.

Das Wundabdeckungselement weist ein öffenbares Fenster 113 auf, das mittels eines gasdichten Leistenverschlusses 114 an dem Wundabdeckungselement angeordnet ist. Der gezeigte Leistenverschluss ist auch unter den Pseudonymen "Ziplock", "Minigrip" oder Gleitverschluss bekannt. Er weist eine geringe Bauhöhe und ein geringes Gewicht auf und erfordert überdies eine geringe Kraft beim Schließen bzw. Öffnen.

Unterhalb des öffenbares Fensters 113 ist ein Netz 115 vorgesehen, dass Raum für einen nicht dargestellten Absorptionskörper - beispielsweise aufweisend Superabsorbierende Polymere - bietet. Besagter Absorptionskörper kann in das Netz eingelegt werden, woraufhin das öffenbare Fenster dann mittels des Leistenverschlusses an dem Abdeckungselement befestigt werden kann. Nach erneutem Öffnen des Fensters kann der Absorptionskörper entnommen und entsorgt bzw. ausgetauscht werden, beispielsweise dann, wenn er seine volle Aufnahmekapazität erreicht hat.

Ferner ist wundseitig ein weiterer Absorptionskörper 116 vorgesehen, der ggf. eine geringere Flüssigkeitsretention aufweist als der zuvor genannte Absorptionskörper.

Fig. 11b zeigt eine ähnliche Vorrichtung wie Fig. 11a, mit dem Unterschied, dass wundseitig an dem öffenbaren Fenster 117 ein Absorptionskörper 118 befestigt ist. Hat letzterer seine volle Aufnahmekapazität erreicht, kann das gesamte Fenster mitsamt dem daran befestigten Absorptionskörper entsorgt und gegen ein neues ausgetauscht werden.

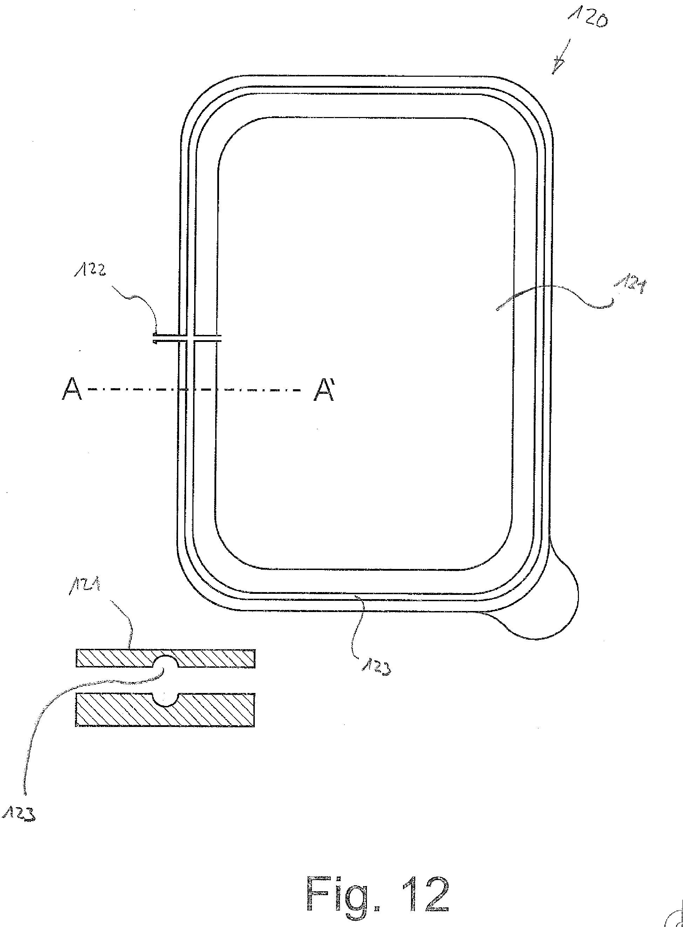

Fig. 12 zeigt eine Wundpflegevorrichtung 120 zur Behandlung von Wunden mittels atmosphärischem Unterdruck im Wundbereich, aufweisend ein Wundabdeckungselement, das an der Haut eines Patienten befestigbar ist, sowie eine Anschlussvorrichtung 122 zum Ansaugen von fluiden Medien. Das Wundabdeckungselement weist ein öffenbares Fenster 121 auf, das mittels eines umlaufenden evakuierbaren Kanals 123 an dem Wundabdeckungselement angeordnet ist.

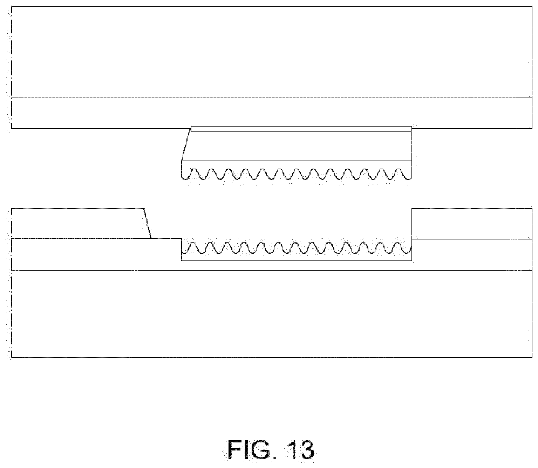

Fig. 13 zeigt eine weitere Ausgestaltung eines gasdichten Verschluss in Form eines Siegel/Klebeverschlusses.

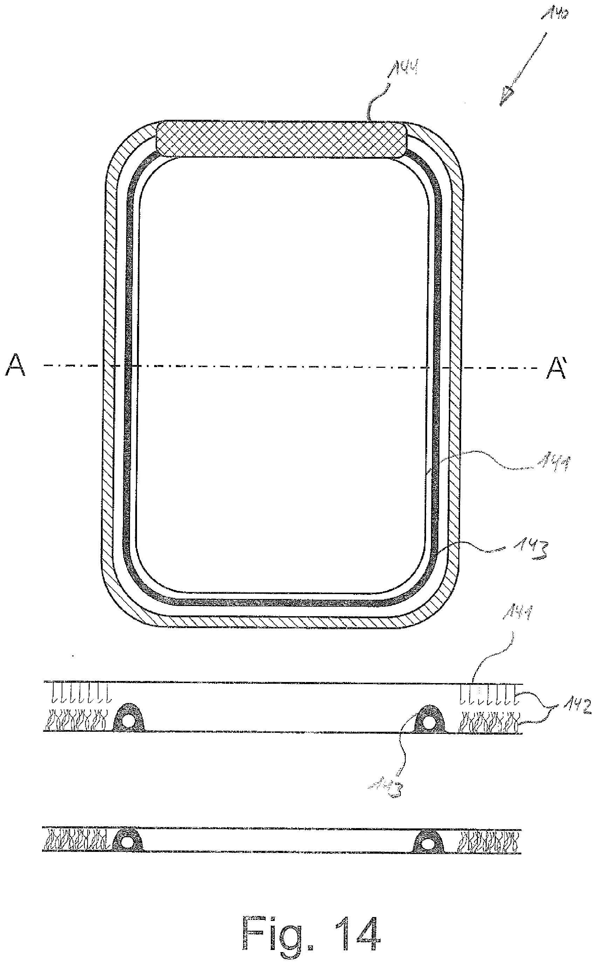

Fig. 14 zeigt eine Wundpflegevorrichtung 140 zur Behandlung von Wunden mittels atmosphärischem Unterdruck im Wundbereich, aufweisend ein Wundabdeckungselement, das an der Haut eines Patienten befestigbar ist, sowie eine nicht dargestellte Anschlussvorrichtung zum Ansaugen von fluiden Medien.

Das Wundabdeckungselement weist ein öffenbares Fenster 141 auf, das mittels eines Klettverschlusses 142 an dem Wundabdeckungselement befestigt werden kann. Mittig ist weiterhin ein umlaufendes Schlauchgummi 143 vorgesehen, dass durch den Klettverschluss mit Druck beaufschlagt wird und so zu einer gasdichten Abdichtung führt. Alternativ zu dem Schlauchgummi kann auch ein Dichtungsgummi, ein Korkband oder ähnliches vorgesehen sein.

Ferner ist ein Scharnier 144 vorgesehen, das das öffenbare Fenster an einer Seite mit dem Wundabdeckungselement verbindet, so dass letzteres nur aufgeklappt werden kann, nicht jedoch in seiner Gänze abgenommen.

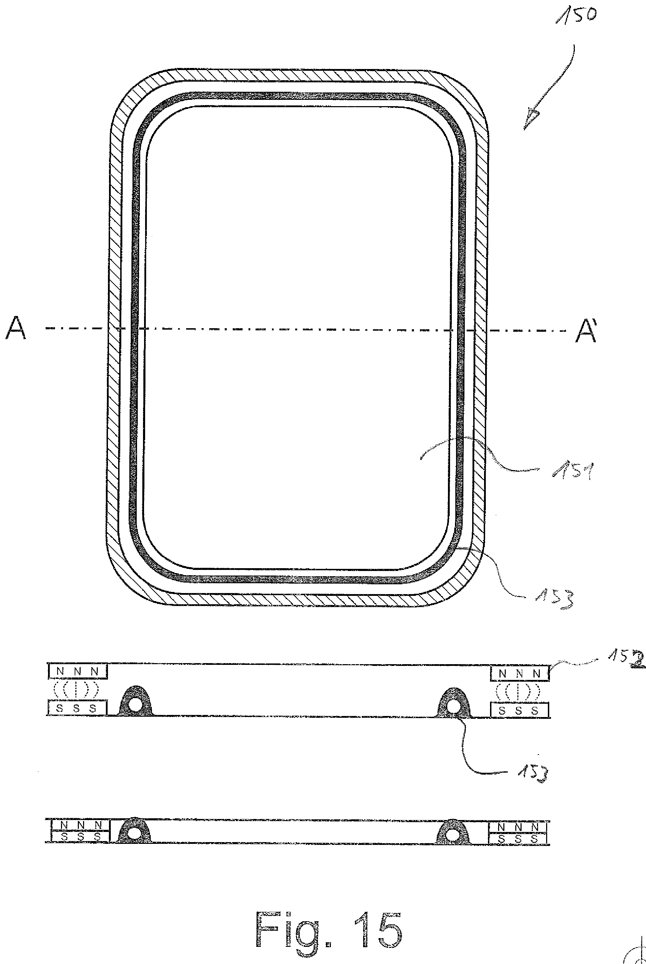

Fig. 15 zeigt eine Wundpflegevorrichtung 140 zur Behandlung von Wunden mittels atmosphärischem Unterdruck im Wundbereich, aufweisend ein Wundabdeckungselement, das an der Haut eines Patienten befestigbar ist, sowie eine nicht dargestellte Anschlussvorrichtung zum Ansaugen von fluiden Medien.

Das Wundabdeckungselement weist ein öffenbares Fenster 151 auf, das mittels eines Magnetverschlusses 152 an dem Wundabdeckungselement befestigt werden kann. Mittig ist weiterhin ein umlaufendes Schlauchgummi 153 vorgesehen, dass durch den Klettverschluss mit Druck beaufschlagt wird und so zu einer gasdichten Abdichtung führt. Alternativ zu dem Schlauchgummi kann auch ein Dichtungsgummi, ein Korkband oder ähnliches vorgesehen sein.

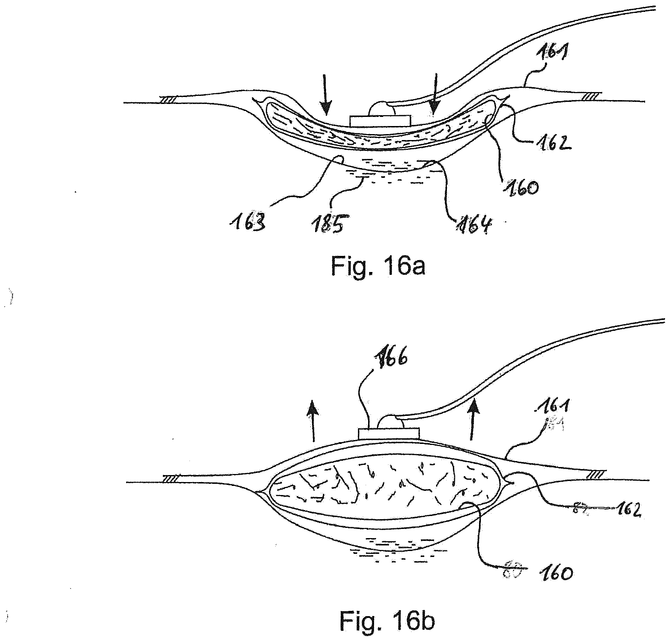

Fig. 16a zeigt die Anordnung des erfindungsgemäßen Wundpflegeartikels mit einem Absorptionskörper 80 mit einem elastischen folienartigen Element 81 und/oder einer elastischen Hülle 82 an einer tiefen Wunde 83, an deren Wundgrund 84 Exsudat 85 steht. Abweichend von Fig. 8 kann die Hülle 82 mindestens abschnittsweise - beispielsweise im wundabgewandten Bereich - ein integraler Bestandteil des folienartigen Elements sein. Durch das elastische folienartige Element 81 und/oder die elastische Hülle 82 ist gewährleistet, dass bei Anlegen eines Unterdrucks der Absorptionskörper an den Wundgrund 84 herangezogen bzw. herangepresst werden kann (angedeutet durch die Pfeile), was insbesondere bei tiefen Wunden erforderlich ist, um so den Kontakt mit dem aufzunehmenden Exsudat 85 herzustellen.

Fig. 16b zeigt eine ähnliche Anordnung wie Fig. 16a, jedoch zu einem späteren Zeitpunkt. Der Absorptionskörper 80 hat bereits große Mengen an Exsudat aufgenommen. Durch das elastische folienartige Element 81 und/oder die elastische Hülle 82 ist gewährleistet, letztere sich der durch die Flüssigkeitsaufnahme bedingten Expansion des Absorptionskörpers nicht entgegenstellen. So wird gewährleistet, dass der Absorptionskörper seine volle Aufnahmekapazität ausspielen kann. Zu diesem Zeitpunkt kann die Kupplungseinrichtung 86 (und damit die nicht dargestellte, daran angeschlossene Unterdruckvorrichtung) bereits abgekoppelt sein, oder aber die Unterdruckvorrichtung ist abgestellt und zieht keinen Unterdruck mehr.

Es kann also vorgesehen sein, dass das Anlegen eines Unterdrucks lediglich dazu dient, den Absorptionskörper an den Wundgrund 84 heranzuziehen bzw. heranzupressen, um so den Kontakt mit dem aufzunehmenden Exsudat herzustellen. Sobald dieser Kontakt hergestellt ist, kann vorgesehen sein, die Vakuumvorrichtung abzutrennen bzw. abzuschalten.

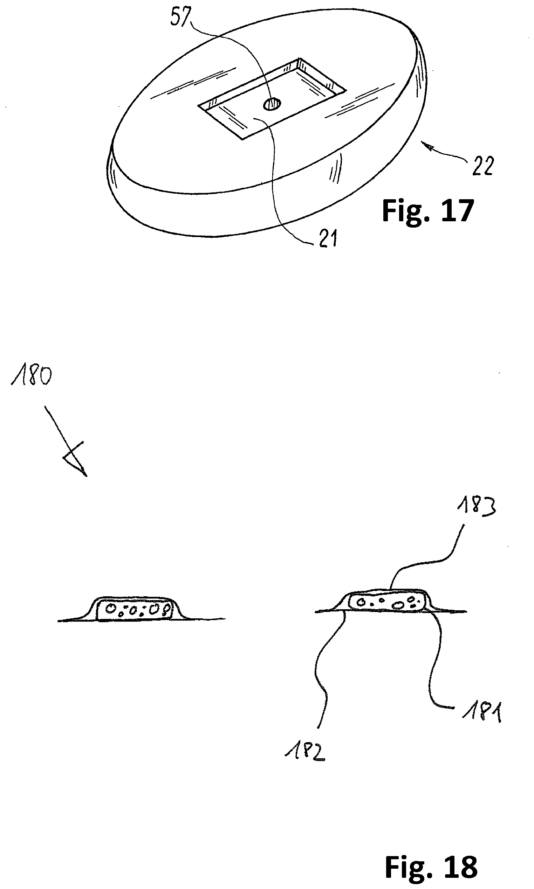

Eine weitere Ausführungsform ist der Fig. 17 zu entnehmen. Hierbei ist ein Schaumstoffkörper vorgesehen, der eine Aussparung für die passgenaue Aufnahme einer Unterdruckquelle, beispielsweise einer Pumpe aufweist. In einer durchgehenden Öffnung 57 ist bevorzugt ein nicht dargestelltes Rückschlagventil platziert. Besagter Schaumstoffkörper von dem nicht gezeigten Wundabdeckungselement abgedeckt.

Fig. 18 zeigt einen Querschnitt durch einen Rahmen 180 eines erfindungsgemäßen Wundabdeckungselements. Der Rahmen besteht beispielsweise aus einem Schaumstoffmaterial 181, das einlaminiert ist in eine gasdichte Grundfolie 182 und eine darauf aufkaschierte gasdichte Abdeckfolie 183. Erstere bildet die Kontaktfläche zur Haut des Patienten und ist beispielsweise mit einem Kleber beschichtet wie andernorts hierin beschichtet. Letztere bildet die Auflagefläche für das öffenbare Fenster. Sie kann bevorzugt desinfizierbar ausgestaltet sein.

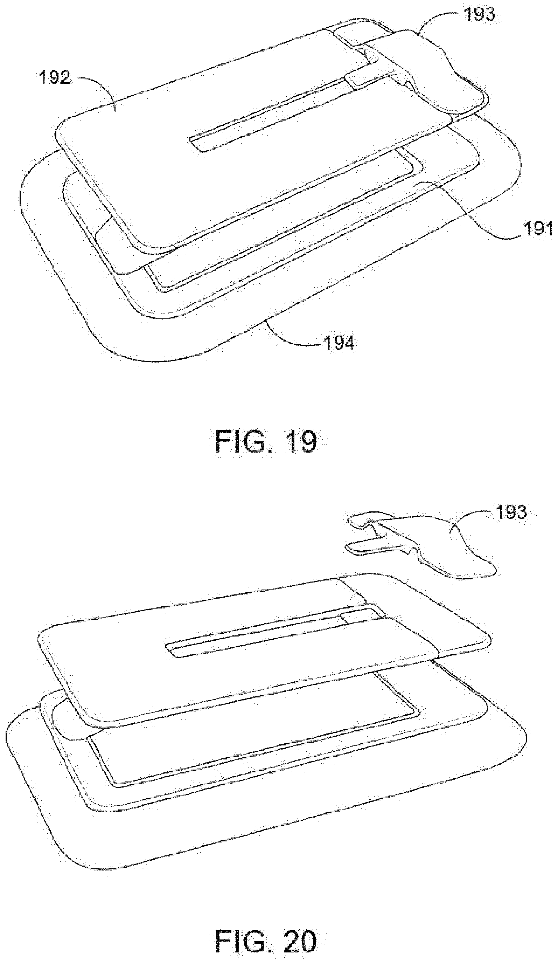

Figs. 19 - 22 zeigen konkretisierte Ausführungsformen der erfindungsgemäßen Wundpflegevorrichtung.

Fig. 19 zeigt eine Wundpflegevorrichtung zur Behandlung von Wunden mittels atmosphärischem Unterdruck im Wundbereich, aufweisend ein Wundabdeckungselement 191 in Form eines Rahmens, das an der Haut eines Patienten über eine unterlegte Folie 194 beispielsweise mit Hilfe eines physiologisch unbedenklichen Klebers befestigbar ist, wobei das Wundabdeckungselement ein öffenbares Fenster 192 aufweist, das mittels eines gasdichten Verschlusses - beispielsweise eine Beschichtung aus Low-Adhesive-Silikon - an dem Wundabdeckungselement angeordnet ist. Das Wundabdeckungselement beispielsweise ein in ein gasdichtes Material einkaschiertes Schaumstoffmaterial oder ein Abstandsgewirke auf. Das Fenster weist eine Unterlegung auf, die beispielsweise ein Schaumstoffmaterial oder ein Abstandsgewirke aufweist, das von einer gasdichten Folie oder Beschichtung abgeschlossen wird. Ferner ist eine Pumpe 193 dargestellt, die über eine Anschluss Vorrichtung zum Ansaugen von fluiden Medien an die Wundpflegevorrichtung angeschlossen ist. Das öffenbare Fenster 192 weist eine Aussparung in seinem Material für die passgenaue Aufnahme einer der Pumpe 193 auf.

Die Pumpe 193 ist abnehmbar ausgebildet, wie in Fig. 20 zu erkennen.

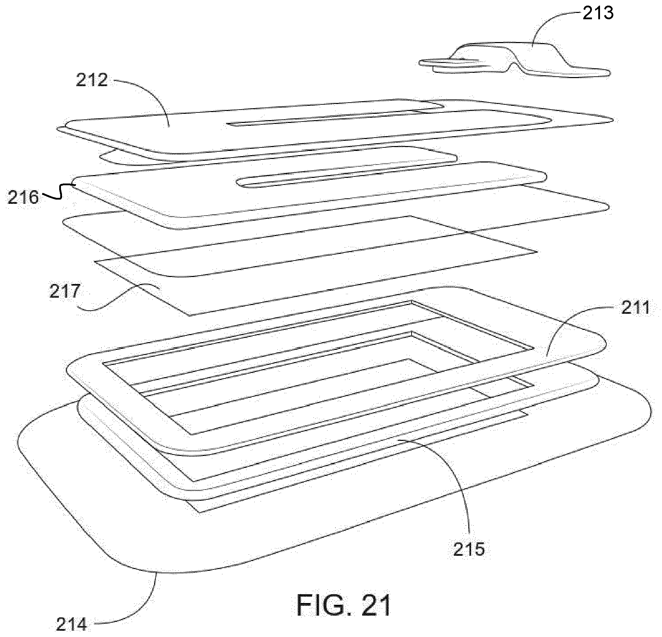

Fig. 21 zeigt die Wundpflegevorrichtung in Explosionsansicht mit dem Wundabdeckungselement 211 in Form eines Rahmens, das an der Haut eines Patienten über eine unterlegte Folie 214 beispielsweise mit Hilfe eines physiologisch unbedenklichen Klebers befestigbar ist. Ebenso ist gezeigt das Schaumstoffmaterial oder Abstandsgewirke 215 des Wundabdeckungselement 211.

Fig. 21 zeigt ferner das öffenbare Fenster 212 aufweist, das mittels eines gasdichten Verschlusses - beispielsweise eine Beschichtung aus Low-Adhesive-Silikon - an dem Wundabdeckungselement angeordnet ist, sowie das dem Fenster unterlegte Schaumstoffmaterial oder Abstandsgewirke aufweist 216, das von einer gasdichten Folie oder Beschichtung abgeschlossen wird.

Ferner ist die Pumpe 213, die über eine Anschlussvorrichtung zum Ansaugen von fluiden Medien an die Wundpflegevorrichtung angeschlossen ist, erkennbar, sowie die Aussparung in im Fenster zur passgenauen Aufnahme einer Pumpe.

Weiterhin ist ein Wunddistanzgitter 217 dargestellt, dass entweder (i) wundseitig vorgesehen sein, und sowohl lose innerhalb des Rahmen anordenbar sein als auch am Rahmen fixiert sein kann, oder (ii) deckelseitig vorgesehen sein kann.

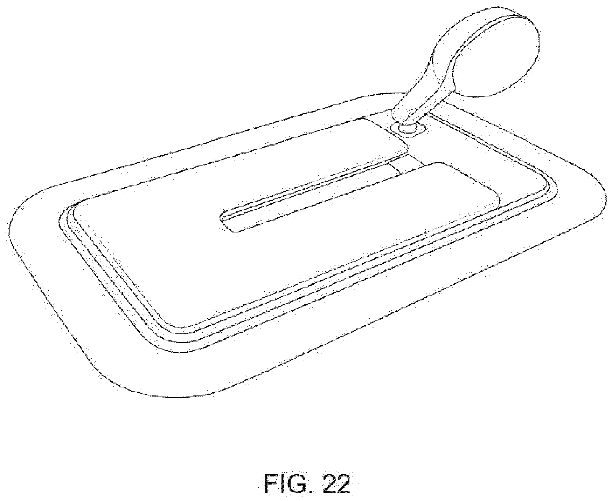

Fig. 22 zeigt eine weitere Wundpflegevorrichtung zur Behandlung von Wunden mittels atmosphärischem Unterdruck im Wundbereich, wobei hier die Pumpe als handbetriebene Pumpe ausgebildet ist.

## Patentansprüche

1. Wundpflegevorrichtung (60, 70, 80, 90, 100, 110, 120, 140, 150) zur Behandlung von Wunden mittels atmosphärischem Unterdruck im Wundbereich, aufweisend ein Wundabdeckungselement (3, 61, 71, 91, 101, 111, 191, 211), das an der Haut eines Patienten befestigbar ist sowie eine Anschlussvorrichtung zum Ansaugen von fluiden Medien (62, 82, 92, 102, 112, 122), wobei das Wundabdeckungselement ein öffenbares Fenster (10, 23, 63, 73, 83, 93, 103, 113, 117, 121, 141, 151, 192, 212) mit Fensterdeckel 44 aufweist, das mittels eines gasdichten Verschlusses an dem Wundabdeckungselement angeordnet ist, **dadurch gekennzeichnet, dass** die Vorrichtung ferner einen Wundexsudate (165) absorbierenden Absorptionskörper (84, 95, 105, 116, 118, 160) aufweist, der mindestens eine superabsorbierende Substanz aufweist, und wobei die Wundpflegevorrichtung ein deckelseitig vorgesehenes Wunddistanzgitter zur Verhinderung des Gelblockings aufweist.

2. Wundpflegevorrichtung (60, 70, 80, 90, 100, 110, 120, 140, 150) gemäß Anspruch 1, **dadurch gekennzeichnet, dass** besagtes öffenbares Fenster eine elastische Rückwand aufweist, die ein Silikonmaterial aufweist, oder auf der Unterseite mit einem Silikonmaterial beschichtet ist oder mit einem Silikon-Material unterlegt ist.

3. Wundpflegevorrichtung (60, 70, 80, 90, 100, 110, 120, 140, 150) gemäß Anspruch 1 oder 2, **dadurch gekennzeichnet, dass** besagter gasdichter Verschluss in Form eines Klettverschlusses mit innenliegender Dichtungslippe ausgeführt ist.

4. Wundpflegevorrichtung (60, 70, 80, 90, 100, 110, 120, 140, 150) gemäß einem der vorhergehenden Ansprüche, wobei das Wundabdeckungselement (3, 61, 71, 91, 101, 111, 191, 211) dem anatomischen Relief einer gegebenen Körperposition entsprechend vorgeformt ist.

5. Wundpflegevorrichtung (60, 70, 80, 90, 100, 110, 120, 140, 150) gemäß einem der vorhergehenden Ansprüche, wobei das Wundabdeckungselement (3, 61, 71, 91, 101, 111, 191, 211) dergestalt ausgebildet ist, dass es der, durch den Bewegungsablauf gegebenen Körperposition folgen kann.

6. Wundpflegevorrichtung (60, 70, 80, 90, 100, 110, 120, 140, 150) gemäß einem der vorhergehenden Ansprüche, wobei das Wundabdeckungselement (3, 61, 71, 91, 101, 111, 191, 211) mithilfe eines adhäsiven Materials (65) an der Haut eines Patienten befestigbar ist, wobei letzteres ausgewählt ist aus der Gruppe enthaltend
• Acrylatkleber
• Silikon
• Hydrokolloidkleber
• Zinkoxid-Kleber, und/oder
• Latexkleber.

7. Wundpflegevorrichtung (60, 70, 80, 90, 100, 110, 120, 140, 150) gemäß einem der vorhergehenden Ansprüche, **dadurch gekennzeichnet, dass** der Absorptionskörper (84, 95, 105, 116, 118, 160) eine modifizierte Cellulose, einen Schaumstoff und/oder ein Alginat aufweist und bevorzugt ferner ein Vlies aufweisend Zellulosefasern aufweist.

8. Wundpflegevorrichtung (60, 70, 80, 90, 100, 110, 120, 140, 150) gemäß einem der vorhergehenden Ansprüche, **dadurch gekennzeichnet, dass** im Bereich der Anschlussvorrichtung zum Ansaugen von fluiden Medien (62, 82, 92, 102, 112, 122) eine Barriere angeordnet ist, die keine Flüssigkeiten passieren lasst.

9. Wundpflegevorrichtung (60, 70, 80, 90, 100, 110, 120, 140, 150) gemäß einem der vorhergehenden Ansprüche, **dadurch gekennzeichnet, dass** die Vorrichtung ferner eine Einrichtung zur Erzeugung von atmosphärischem Unterdruck (193, 213) aufweist, die bevorzugt unmittelbar an der Wundpflegevorrichtung angeordnet ist.

10. Wundpflegevorrichtung (60, 70, 80, 90, 100, 110, 120, 140, 150) gemäß einem der vorhergehenden Ansprüche, **dadurch gekennzeichnet, dass** zwischen der Einrichtung zur Erzeugung von atmosphärischem Unterdruck und dem Wundabdeckungselement (3, 61, 71, 91, 101, 111, 191, 211) bzw. dem Wundexsudate aufsaugenden Absorptionskörper eine Kupplung, ein Absperrventil und/oder ein Dreiwegehahn angeordnet ist.

11. Wundpflegevorrichtung (60, 70, 80, 90, 100, 110, 120, 140, 150) gemäß einem der vorhergehenden Ansprüche, **dadurch gekennzeichnet, dass** der Absorptionskörper (84, 95, 105, 116, 118, 160) mit einer flüssigkeitspermeablen Hülle (162) umgeben ist.

12. Wundpflegevorrichtung (60, 70, 80, 90, 100, 110, 120, 140, 150) gemäß einem der vorhergehenden Ansprüche, **dadurch gekennzeichnet, dass** die Vorrichtung ferner einen Abstandskörper aufweist und/oder ferner eine Lage aufweisend ein Schwermetall, bevorzugt Kupfer oder Silber, oder ein Salz desselben, aufweist.

13. Wundpflegevorrichtung (60, 70, 80, 90, 100, 110, 120, 140, 150) gemäß einem der vorhergehenden Ansprüche, **dadurch gekennzeichnet, dass** das Wundabdeckungselement (3, 61, 71, 91, 101, 111, 191, 211) flüssigkeits- und/oder gasundurchlässig ist und bevorzugt wasserdampfdurchlässig ist.

## Claims

1. Wound care device (60, 70, 80, 90, 100, 110, 120, 140, 150) for treating wounds by means of atmospheric negative pressure in the wound region, comprising a wound covering element (3, 61, 71, 91, 101, 111, 191, 211) which can be attached to the skin of a patient, and a connection device for suctioning of fluid media (62, 82, 92, 102, 112, 122), the wound covering element comprising an openable window (10, 23, 63, 73, 83, 93, 103, 113, 117, 121, 141, 151, 192, 212) having a window cover 44, which window is arranged on the wound covering element by means of a gas-tight closure, **characterized in that** the device further comprises an absorption body (84, 95, 105, 116, 118, 160) which absorbs wound exudate (165) and which comprises at least one superabsorbent substance, and wherein the wound care device comprises a wound spacer mesh provided on the cover side to prevent gel blocking.

2. Wound care device (60, 70, 80, 90, 100, 110, 120, 140, 150) according to claim 1, **characterized in that** said openable window comprises an elastic back wall which comprises a silicone material, or is coated on the underside with a silicone material or is lined with a silicone material.

3. Wound care device (60, 70, 80, 90, 100, 110, 120, 140, 150) according to either claim 1 or claim 2, **characterized in that** said gas-tight closure is in the form of a hook-and-loop fastener having an internal sealing lip.

4. Wound care device (60, 70, 80, 90, 100, 110, 120, 140, 150) according to any of the preceding claims, wherein the wound covering element (3, 61, 71, 91, 101, 111, 191, 211) is preformed so as to correspond to the anatomical relief of a given body position.

5. Wound care device (60, 70, 80, 90, 100, 110, 120, 140, 150) according to any of the preceding claims, wherein the wound covering element (3, 61, 71, 91, 101, 111, 191, 211) is designed in such a way that it can follow the body position given by the movement sequence.

6. Wound care device (60, 70, 80, 90, 100, 110, 120, 140, 150) according to any of the preceding claims, wherein the wound covering element (3, 61, 71, 91, 101, 111, 191, 211) can be attached to the skin of a patient by means of an adhesive material (65), the latter being selected from the group containing
• acrylic adhesive
• silicone
• hydrocolloid adhesive
• zinc oxide adhesive, and/or
• latex adhesive.

7. Wound care device (60, 70, 80, 90, 100, 110, 120, 140, 150) according to any of the preceding claims, **characterized in that** the absorption body (84, 95, 105, 116, 118, 160) comprises a modified cellulose, a foam and/or an alginate, and preferably further comprises a nonwoven material comprising cellulose fibers.

8. Wound care device (60, 70, 80, 90, 100, 110, 120, 140, 150) according to any of the preceding claims, **characterized in that** a barrier is arranged in the region of the connection device for suctioning fluid media (62, 82, 92, 102, 112, 122), which barrier does not allow any liquids to pass through.

9. Wound care device (60, 70, 80, 90, 100, 110, 120, 140, 150) according to any of the preceding claims, **characterized in that** the device further comprises an apparatus for generating atmospheric negative pressure (193, 213), which is preferably arranged directly on the wound care device.

10. Wound care device (60, 70, 80, 90, 100, 110, 120, 140, 150) according to any of the preceding claims, **characterized in that** a coupling, a shut-off valve and/or a three-way valve is arranged between the apparatus for generating atmospheric negative pressure and the wound covering element (3, 61, 71, 91, 101, 111, 191, 211) or the absorption body which absorbs wound exudate.

11. Wound care device (60, 70, 80, 90, 100, 110,120, 140, 150) according to any of the preceding claims, **characterized in that** the absorption body (84, 95, 105, 116, 118, 160) is surrounded by a liquid-permeable sheath (162).

12. Wound care device (60, 70, 80, 90, 100, 110, 120, 140, 150) according to any of the preceding claims, **characterized in that** the device further comprises a spacer body and/or further comprises a layer comprising a heavy metal, preferably copper or silver, or a salt thereof.

13. Wound care device (60, 70, 80, 90, 100, 110, 120, 140, 150) according to any of the preceding claims, **characterized in that** the wound covering element (3, 61, 71, 91, 101, 111, 191, 211) is impermeable to liquid and/or gas and is preferably permeable to water vapor.

## Revendications

1. Dispositif de soin de plaies (60, 70, 80, 90, 100, 110, 120, 140, 150) destiné à traiter des plaies à l'aide d'une dépression atmosphérique dans la zone de plaie, comprenant un élément de recouvrement de plaie (3, 61, 71, 91, 101, 111, 191, 211) qui peut être fixé à la peau d'un patient, ainsi qu'un dispositif de raccordement destiné à aspirer des milieux fluides (62, 82, 92, 102, 112, 122), dans lequel l'élément de recouvrement de plaie comprend une fenêtre ouvrable (10, 23, 63, 73, 83, 93, 103, 113, 117, 121, 141, 151, 192, 212) comportant un couvercle de fenêtre (44), laquelle fenêtre est disposée sur l'élément de recouvrement de plaie à l'aide d'une fermeture étanche aux gaz, **caractérisé en ce que** le dispositif comprend en outre un corps d'absorption (84, 95, 105, 116, 118, 160) absorbant des exsudats de plaie (165), lequel corps d'absorption comprend au moins une substance superabsorbante, et dans lequel le dispositif de soin de plaies comprend une grille d'écartement de plaie prévue côté couvercle et destinée à empêcher le blocage de gel.

2. Dispositif de soin de plaies (60, 70, 80, 90, 100, 110, 120, 140, 150) selon la revendication 1, **caractérisé en ce que** ladite fenêtre ouvrable comprend une paroi arrière élastique qui comprend un matériau en silicone, ou est revêtue d'un matériau en silicone sur le côté inférieur ou est doublée d'un matériau en silicone.

3. Dispositif de soin de plaies (60, 70, 80, 90, 100, 110, 120, 140, 150) selon la revendication 1 ou 2, **caractérisé en ce que** ladite fermeture étanche aux gaz est réalisée sous la forme d'une fermeture autoagrippante comportant une lèvre d'étanchéité interne.

4. Dispositif de soin de plaies (60, 70, 80, 90, 100, 110, 120, 140, 150) selon l'une des revendications précédentes, dans lequel l'élément de recouvrement de plaie (3, 61, 71, 91, 101, 111, 191, 211) est préformé en fonction du relief anatomique d'une position corporelle donnée.

5. Dispositif de soin de plaies (60, 70, 80, 90, 100, 110, 120, 140, 150) selon l'une des revendications précédentes, dans lequel l'élément de recouvrement de plaie (3, 61, 71, 91, 101, 111, 191, 211) est conçu de telle sorte qu'il peut suivre la position corporelle, laquelle est donnée par la série de mouvements.

6. Dispositif de soin de plaies (60, 70, 80, 90, 100, 110, 120, 140, 150) selon l'une des revendications précédentes, dans lequel l'élément de recouvrement de plaie (3, 61, 71, 91, 101, 111, 191, 211) peut être fixé à la peau d'un patient au moyen d'un matériau adhésif (65), dans lequel ce dernier est choisi dans le groupe contenant
• colle acrylate
• silicone
• colle hydrocolloïde
• colle à l'oxyde de zinc, et/ou
• colle au latex.

7. Dispositif de soin de plaies (60, 70, 80, 90, 100, 110, 120, 140, 150) selon l'une des revendications précédentes, **caractérisé en ce que** le corps d'absorption (84, 95, 105, 116, 118, 160) comprend une cellulose modifiée, une mousse et/ou un alginate, et comprend en outre de préférence un non-tissé comprenant des fibres de cellulose.

8. Dispositif de soin de plaies (60, 70, 80, 90, 100, 110, 120, 140, 150) selon l'une des revendications précédentes, **caractérisé en ce que**, dans la zone du dispositif de raccordement destiné à aspirer des milieux fluides (62, 82, 92, 102, 112, 122), est disposée une barrière qui ne laisse passer aucun liquide.

9. Dispositif de soin de plaies (60, 70, 80, 90, 100, 110, 120, 140, 150) selon l'une des revendications précédentes, **caractérisé en ce que** le dispositif comprend en outre un appareil destiné à générer une dépression atmosphérique (193, 213), lequel appareil est de préférence disposé directement sur le dispositif de soin de plaies.

10. Dispositif de soin de plaies (60, 70, 80, 90, 100, 110, 120, 140, 150) selon l'une des revendications précédentes, **caractérisé en ce qu'**un accouplement, une soupape d'arrêt et/ou un robinet à trois voies sont disposés entre l'appareil destiné à générer une dépression atmosphérique et l'élément de recouvrement de plaie (3, 61, 71, 91, 101, 111, 191, 211) ou le corps d'absorption aspirant des exsudats de plaie.

11. Dispositif de soin de plaies (60, 70, 80, 90, 100, 110, 120, 140, 150) selon l'une des revendications précédentes, **caractérisé en ce que** le corps d'absorption (84, 95, 105, 116, 118, 160) est entouré d'une enveloppe perméable aux liquides (162).

12. Dispositif de soin de plaies (60, 70, 80, 90, 100, 110, 120, 140, 150) selon l'une des revendications précédentes, **caractérisé en ce que** le dispositif comprend en outre un corps d'espacement et/ou comprend en outre une couche comprenant un métal lourd, de préférence du cuivre ou de l'argent, ou un sel de celui-ci.

13. Dispositif de soin de plaies (60, 70, 80, 90, 100, 110, 120, 140, 150) selon l'une des revendications précédentes, **caractérisé en ce que** l'élément de recouvrement de plaie (3, 61, 71, 91, 101, 111, 191, 211) est imperméable aux liquides et/ou aux gaz et est de préférence perméable à la vapeur d'eau.
